# FASCICULE DE BREVET EUROPEEN

(11) **EP 1 098 903 B1**
(45) Date de publication et mention de la délivrance du brevet: **03.09.2008**
(21) Numéro de dépôt: 99957167.2
(22) Date de dépôt: 21.06.1999
(51) Int. Cl.: C07K 7/64, A61K 38/13

(54) **PROCEDE DE PREPARATION DE DERIVES DE CYCLOSPORINE**
VERFAHREN ZUR HERSTELLUNG VON CYCLOSPORIN-DERIVATEN
METHOD FOR PREPARING CYCLOSPORIN DERIVATIVES

(30) Priorité: 22.06.1998 FR 9807846
(43) Date de publication de la demande: 16.05.2001
(73) Titulaire: Aventis Pharma S.A., 92160 Antony (FR)
(72) Inventeur: VISKOV, Christian, F-91130 Ris Orangis (FR)
(74) Mandataire: Rauline, Mathilde
(86) Numéro de dépôt international: PCT/FR1999/001480
(87) Numéro de publication internationale: WO 1999/067280

(56) Documents cités:
- EP-A- 0 194 972
- EP-A- 0 357 428
- EP-A- 0 379 063
- EP-A- 0 484 281
- WO-A-99/32512
- SEEBACH D ET AL: "MODIFICATION OF CYCLOSPORIN A (CS): GENERATION OF AN ENOLATE AT THESARCOSINE RESIDUE AND REACTIONS WITH ELECTROPHILES" HELVETICA CHIMICA ACTA, vol. 76, no. 4, 1993, pages 1564-1590, XP002022424
- T.L. RATHMAN: "Lithium reagents for syntheses." SPEC. CHEM., vol. 9, no. 5, 1989, pages 300-306, XP002098147
- S.L. BELAGALI ET AL: "A highly efficient method of N-methylation for the amino acid derivatives" INDIAN J. CHEMISTRY, vol. 34B, janvier 1995 (1995-01), pages 45-47, XP002098149
- K.S. CHU ET AL.: "Asymmetric total synthsis of (+)-Jasplakinolide" J. ORGANIC CHEMISTRY, vol. 56, 1991, pages 5196-5202, XP002098148

## Description

La présente invention se rapporte à un nouveau procédé de préparation de dérivés de cyclosporine modifiés en position -3, qui consiste à traiter une cyclosporine avec un sel métallique de l'hexaméthyldisilazane.

Les cyclosporines constituent un groupe d'undécapeptides poly-N-méthylés cycliques qui possèdent dans la majorité des cas des propriétés immunosuppressives, anti-inflammatoires, et anti-parasitaires, mais qui peuvent aussi être non-immunosuppressives et présenter une activité sur le VIH (Virus de l'Immuno-déficience Humaine).

Une des premières cyclosporines naturelles à avoir été isolée est connue sous le nom de cyclosporine A dont la structure est la suivante :

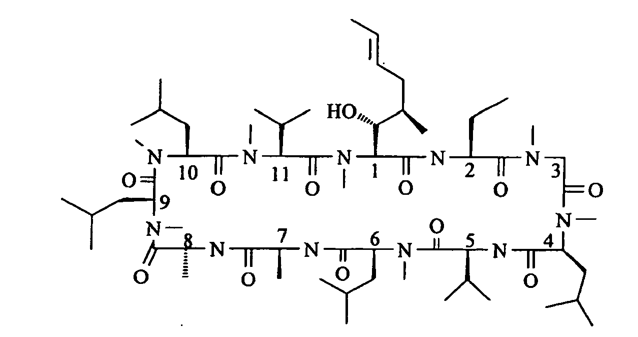

Aujourd'hui, de nombreuses cyclosporines naturelles sont connues et isolées (par exemple les cyclosporines A à Z ci-après dénommées "cyclosporines"), et de nombreuses cyclosporines de synthèse ont été préparées (ci-après dénommées "dérivés de cyclosporine").

Actuellement, les dérivés de cyclosporine modifiés en position -3 sont obtenus selon la méthode décrite par D.Seebach et al dans Helvetica chimica Acta, vol.76, n°4, 1993, p.1564-1590, ainsi que dans le brevet EP 194972 citant le même inventeur. Cette méthode consiste notamment à préparer dans un premier temps l'anion en 3 d'une cyclosporine par action d'une base forte telle que le diisopropylamidure de lithium ou le butyllithium, puis à préparer dans un second temps des dérivés de cyclosporine modifiés en position -3 par addition d'un agent électrophile. Le principal inconvénient de cette méthode réside dans le fait que la réaction nécessite d'être effectuée à très basse température. En outre, le rendement global est généralement extrêmement faible. Par ailleurs, il est nécessaire de travailler en présence d'un très large excès d'électrophile. Par cette méthode, il se forme de plus l'épimère S en quantité importante par rapport à l'épimère R, et une étape de séparation est donc nécessaire.

Aujourd'hui, la demanderesse a montré que le procédé selon l'invention permet d'aboutir à des dérivés de cyclosporine modifiés en position -3 avec un rendement nettement amélioré. Un autre avantage de ce procédé réside dans le fait que la réaction est menée à une température beaucoup moins basse et avec un excès d'électrophile nettement moins important, ce qui facilite la mise en oeuvre à l'échelle industrielle. La demanderesse a également mis en évidence que l'épimère R requis est formé de façon beaucoup plus sélective.

La présente invention est ainsi caractérisée en ce que l'on prépare un polyanion intermédiaire pour la préparation de dérivés de cyclosporine par traitement d'une cyclosporine par un sel métallique de l'hexaméthyldisilazane en présence d'un halogénure de métal.

Le polyanion obtenu a pour formule générale : 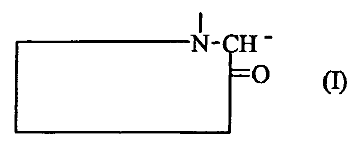 dans laquelle 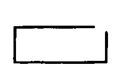 représente une cyclosporine pour laquelle un ou des groupements hydroxy libres et/ou un ou des atomes d'azote non-méthylés en position α et/ou tout autre groupement acide déprotonable sont éventuellement déprotonés ou sous forme protégée.

Généralement, on effectue la réaction dans un éther aliphatique ou cyclique, un hydrocarbure aromatique, ou dans un mélange de ces solvants. A titre d'exemple, on peut citer le tétrahydrofuranne (THF), le t-butylméthyléther (TBME), le diméthoxyéthane (DME), l'anisole, le dioxane ou le toluène. De façon préférée, on opère dans le t-butylméthyléther ou dans le tétrahydrofuranne en présence d'hydrocarbure aromatique.

Le traitement de la cyclosporine est mis en oeuvre à une température comprise entre -40 et 0°C. De façon avantageuse, la réaction est initiée à une température comprise entre -25 et -15°C.

On opère selon l'invention en présence de 20 à 30 équivalents molaires de sel métallique de l'hexaméthyldisilazane, et de préférence entre 23 et 28 équivalents molaires. Le sel métallique de l'hexaméthyldisilazane peut être a titre d'exemple un sel alcalin de l'hexaméthyldisilazane. Notamment, il peut s'agir du sel de lithium de l'hexaméthyldisilazane, du sel de sodium de l'hexaméthyldisilazane, ou encore du sel de potassium de l'hexaméthyldisilazane. Avantageusement, on opère en présence du sel de lithium de l'hexaméthyldisilazane.

Selon une variante de l'invention, la préparation du polyanion de cyclosporine peut être réalisée en présence d'halogénures de métaux. Par exemple, il peut s'agir du chlorure de lithium, du chlorure de césium, du fluorure de césium, du chlorure mercurique, du chlorure cuivreux etc... De façon avantageuse, on opère en présence de 2 à 8 équivalents molaires de chlorure de césium ou de lithium, et plus préférentiellement en présence de 5 à 7 équivalents molaires de chlorure de césium.

Par ailleurs, le ratio (poids/poids) de cyclosporine mise en jeu par rapport au poids total de la solution est généralement inférieur ou égal à 10 %. Préférentiellement, la réaction est effectuée avec un ratio de cyclosporine inférieur ou égal à 6 %. Avantageusement, on introduit entre 2 et 5 % de cyclosporine.

Selon un aspect préféré de la présente invention, le polyanion obtenu a pour formule : 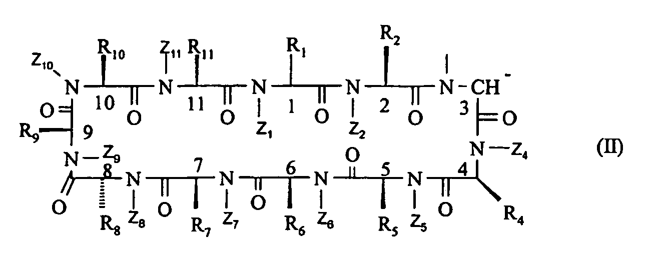 pour laquelle :
i) Les substituants R₁, R₂ et R₄ à R₁₁, et Z₁, Z₂ et Z₄ à Z₁₁ sont définis comme pour la cyclosporine A, ou bien
ii) Les substituants R₁, R₂ et R₄ à R₁₁, et Z₁, Z₂ et Z₄ à Z₁₁ sont définis comme pour la cyclosporine A, à l'exception de R₄ et Z₄ qui sont définis de façon à avoir en position -4 l'acide aminé 4'-(hydroxy)méthylleucine, ou bien
iii) Les substituants R₂, R₅ à R₁₁ et Z₂, Z₅ à Z₁₁ sont définis comme pour la cyclosporine A, et
   - Z₁ est un groupement méthyle, et R₁ a pour formule : 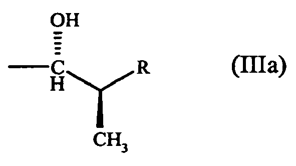
      - R étant un groupement de formule -CH₂-CH=CH-CH₂-R' pour lequel R' représente un radical alkylthio, aminoalkylthio, alkylaminoalkylthio, dialkylaminoalkylthio, pyrimidinylthio, thiazolylthio, N-alkylimidazolylthio, hydroxyalkylphénylthio, hydroxyalkylphényloxy, nitrophénylamino, ou 2-oxopyrimidin-1-yle, ou
      - R étant un groupement de formule -CH₂-S-Alk pour lequel Alk représente un groupement alkyle, et
   - Z₄ et R₄ sont des radicaux tels que l'on a en position -4 un acide aminé méthylleucine (MeLeu) ou 4'-hydroxyMeLeu, ou bien,
iv)
   - Z₁ et R₁ sont des groupements tels que l'on a en position -1 une homothréonine substituée de formule générale :

      Rᵢ-CH₂CH(CH₃)-CH(OH)-CH(NHCH₃)-COOH (IIIb)

      dans laquelle Rᵢ représente le n-propyle ou le propényle et la double liaison présente de préférence une configuration trans, et
   - R₂ et Z₂ sont des radicaux tels que l'on a en position -2 l'acide alpha-aminobutyrique (αAbu), la valine (Val), la norvaline (Nva), ou la thréonine (Thr), et
   - R₄ et Z₄ sont des radicaux tels que l'on a en position -4 la N-méthyl-gamma-hydroxy-leucine ou la N-méthyl-gamma-acétyloxyleucine, et
   - R₅ et Z₅ sont des radicaux tels que l'on a en position -5 la valine, et
   - R₆, Z₆, R₉, Z₉, R₁₀ et Z₁₀ sont des radicaux tels que l'on a en position -6, -9, et-10 la N-méthylleucine, et
   - Z₇ et R₇ sont des radicaux tels que l'on a en position -7 l'alanine (Ala), et
   - Z₈ et R₈ sont des radicaux tels que l'on a en position -8 la (D)-alanine ou la (D)-sérine, et
   - Z₁₁ et R₁₁ sont des radicaux tels que l'on a en position -11 la N-méthyl-valine, ou bien,
v) Z₁ et R₁ sont des groupements tels que l'on a en position -1 un substituant MeBmt ou un substituant ayant pour formule générale : 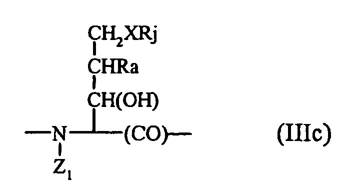 Rⱼ représentant un atome d'hydrogène, ou un groupement alkyle inférieur, un alcényle inférieur, un halogénoalkyle inférieur, un aryle, un alkyloxy inférieur, un alkoxy C₁₋₆alkyle, un hydroxyméthyle, un alkylthio inférieur, un alkylthioC₁₋₆alkyle, un mercaptoC₁₋₆alkyle, ou un hétéroaryle, les groupements aryle et hétéroaryle pouvant être substitués avec un ou plusieurs groupements fonctionnels alkyle C₁₋₆, alcanoyle C₁₋₆, halogénoalkyle C₁₋₆, halogène, cyano, hydroxyalkyle C₁₋₃, alkyloxy C₁₋₆, alkyl-S(O)ₙ C₁₋₆ avec n = 0, 1, ou 2, NR_{b}COR_{c} avec R_{b} et R_{c} représentant indépendamment H ou un alkyle C₁₋₆, -NO₂, -NR_{b}R_{c}, -OR_{b}, -CONR_{b}R_{c}, -COR_{b}, -NR_{b}CONR_{b}R_{c}, NR_{b}COR_{c}, -OCOR_{b}, -SCOR_{b}, ou -OCH₂O-, et
   Rₐ étant un alkyle inférieur, Z₁ étant un alkyle inférieur, un phénylalkyle inférieur, ou un aryle, et X représentant S, SO, SO₂ O, ou bien NR_{b}, et
   - Z₂ et R₂ sont des substituants tels que l'on a en position -2 l'acide aminé L-2-aminobutyryle, norvalyle, L-thréonyle, ou bien le même acide aminé qu'en position -1, et
   - Z₄ et R₄ sont des substituants tels que l'on a en position -4 l'acide aminé N-méthyl-L-leucyle, et
   - Z₅ et R₅ sont des substituants tels que l'on a en position -5 l'acide aminé L-valyle, ou norvalyle, et
   - Z₆ et R₆ sont des substituants tels que l'on a en position -6 l'acide aminé N-méthyl-L-leucyle, et
   - Z₇ et R₇ sont des substituants tels que l'on a en position -7 l'acide aminé L-alanyle, L-2-aminobutyryle, ou L-phénylalanyle, et
   - Z₈ et R₈ sont des substituants tels que l'on a en position -8 l'acide aminé D-alanyle ou L-alanyle, et
   - Z₉ et R₉ sont des substituants tels que l'on a en position -9 l'acide aminé N-méthyl-L-leucyle ou N-méthyl-L-valyle, et
   - Z₁₀ et R₁₀ sont des substituants tels que l'on a en position -10 l'acide aminé N-méthyl-L-leucyle ou L-leucyle, et
   - Z₁₁ et R₁₁ sont des substituants tels que l'on a en position -11 l'acide aminé N-méthyl-L-valyle, L-valyle, ou L-2-aminobutyryle, ou bien
vi) Les substituants R₄ à R₁₁, et Z₄ à Z₁₁ sont définis comme pour la cyclosporine A, et:
   - Z₁ et R₁ sont des substituants tels que l'on a en position -1 l'acide aminé MeBmt ou dihydro-MeBmt, et
   - Z₂ et R₂ sont des substituants tels que l'on a en position -2 l'acide aminé αAbu, Thr, Val, ou Nva, ou bien
vii) Les substituants R₇ à R₁₁, et Z₇ à Z₁₁ sont définis comme pour la cyclosporine A, et :
   - Z₁ et R₁ sont des substituants tels que l'on a en position -1 l'acide aminé MeBmt, dihydro-MeBmt, ou 8'-hydroxy-MeBmt, et
   - Z₂ et R₂ sont des substituants tels que l'on a en position -2 l'acide aminé αAbu, Val, Thr, Nva, ou MeOThr, et
   - Z₄ et R₄ sont des substituants tels que l'on a en position -4 l'acide aminé MeLeu, γ-hydroxy-MeLeu, MeIIe, MeVal, MeThr, MeAla, MeaIle, ou MeaThr, et
   - Z₅ et R₅ sont des substituants tels que l'on a en position -5 l'acide aminé Val, Leu, MeVal, ou MeLeu, et
   - Z₆ et R₆ sont des substituants tels que l'on a en position -6 l'acide aminé MeLeu, γ-hydroxy-MeLeu, ou MeAla,
   à condition que lorsque R₄ et Z₄ signifient MeLeu, R₅ et Z₅ signifient alors MeVal ou MeLeu ou bien R₁ et Z₁ signifient 8'-hydroxy-MeBmt, ou bien
viii) Les substituants R₁, R₂ et R₄ à R₁₁, et Z₁, Z₂ et Z₄ à Z₁₁ définissent une cyclosporine pour laquelle le carbone en 3' du résidu en position -1 ou le carbone en β du résidu en position-2 est substitué par O-acyle ou oxo, et notamment
   - Z₁ et R₁ sont des substituants tels que l'on a en position -1 un résidu de formule générale 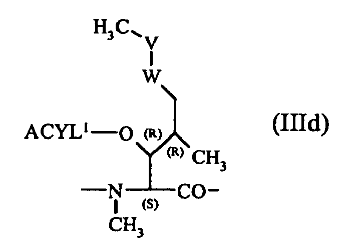 pour laquelle -v-w- est CH₂-CH₂ ou CH=CH trans et ACYL¹ représente un groupe acyle, et
   - Z₂ et R₂ sont des substituants tels que l'on a en position -2 un acide aminé αAbu, Val, Thr, Nva, ou le résidu d'un α-acide aminé β-O-acylé, et
   - Z₅ et R₅ sont des substituants tels que l'on a en position -5 un acide aminé Val, ou Nva lorsque l'on a simultanément un acide aminé Nva en position -2, et
   - Z₈ et R₈ sont des substituants tels que l'on a en position -8 un acide aminé (D)-Ala, un résidu d'un α-acide aminé β-O-acylé ou β-hydroxylé, ayant la configuration (D), et
   - les substituants en position -4, -6, -7, et -9 à -11 sont définis comme pour la cyclosporine A,
   un ou des groupements hydroxy et/ou un ou des atomes d'azote non-méthylés en position α et/ou tout autre groupement acide déprotonable présents dans ladite formule générale (II) étant éventuellement déprotonés ou sous forme protégée.

II est entendu que les positions mentionnées plus haut correspondent à la formule (II).

Les polyanions de formule générale (II) résultants du procédé selon l'invention sont particulièrement intéressants comme intermédiaires pour la préparation de dérivés de cyclosporine de formule générale : 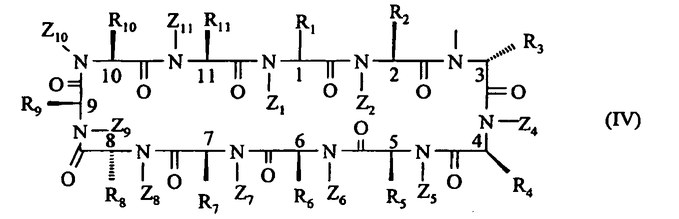 pour laquelle :
1) les substituants R₁, R₂ et R₄ à R₁₁ et Z₁, Z₂, et Z₄, à Z₁₁ sont tels que définis ci-avant en i), et R₃ représente un substituant -S-Alk-R° pour lequel :
   - Alk représente un radical alkylène contenant 2 à 6 atomes de carbone en chaîne droite ou ramifiée ou cycloalkylène contenant 3 à 6 atomes de carbone et
   - R° représente
      - soit un radical carboxy ou alkyloxycarbonyle,
      - soit un radical -NG₁G₂ pour lequel G₁ et G₂, identiques ou différents, représentent des atomes d'hydrogène, ou des radicaux alkyle, alcényle (2 à 4C), cycloalkyle (3 à 6C), phényle éventuellement substitué (par un atome d'halogène, alkyloxy, alkyloxycarbonyle, amino, alkylamino ou dialkylamino), ou représentent des radicaux benzyle ou hétérocyclyle saturé ou insaturé contenant 5 ou 6 chaînons et 1 à 3 hétéroatomes, ou pour lequel G₁ et G₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alkyle, phényle ou benzyle,
      - soit un radical de formule générale : 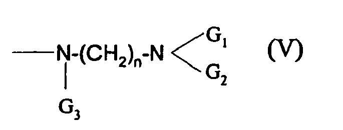 pour lequel G₁ et G₂ sont définis comme ci-dessus, G₃ représente un atome d'hydrogène ou un radical alkyle et n est un nombre entier de 2 à 4, les portions ou radicaux alkyle définis ci-dessus étant droits ou ramifiés et contenant 1 à 4 atomes de carbone, ou bien
2) les substituants R₁, R₂ et R₄ à R₁₁ et Z₁, Z₂, et Z₄ à Z₁₁ sont tels que définis ci-avant en ii), et R₃ représente -S-CH₃ ou un substituant -S-Alk-R° pour lequel :
   - Alk représente un radical alkylène contenant 2 à 6 atomes de carbone en chaîne droite ou ramifiée ou cycloalkylène contenant 3 à 6 atomes de carbone et
   - R° représente
      - soit un radical hydroxy, carboxy ou alkyloxycarbonyle,
      - soit un radical -NG₁G₂ ou un radical de formule générale : 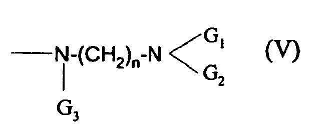 tels que définis ci-avant,
3) les substituants R₁, R₂ et R₄ à R₁₁ et Z₁, Z₂, et Z₄ à Z₁₁ sont tels que définis ci-avant en iii), et R₃ est un radical de structure -S-Alk-R° pour lequel :
   - Alk représente un radical alkylène contenant 2 à 6 atomes de carbone en chaîne droite ou ramifiée ou cycloalkylène contenant 3 à 6 atomes de carbone et
   - R° représente
      - soit un atome d'hydrogène, un radical hydroxy, carboxy ou alkyloxycarbonyle,
      - soit un radical -NG₁G₂ pour lequel G₁ et G₂ identiques ou différents représentent des atomes d'hydrogène, ou des radicaux alkyle, cycloalkyle (3 à 6C), phényle éventuellement substitué (par un atome d'halogène, alkyloxy, alkyloxycarbonyle, amino, alkylamino ou dialkylamino), ou représentent des radicaux benzyle ou hétérocyclyle saturé ou insaturé contenant 5 ou 6 chaînons et 1 à 3 hétéroatomes, ou pour lequel G₁ et G₂ forment avec l'atome d'azote auquel ils sont rattachés un hétérocycle à 5 ou 6 chaînons pouvant contenir un autre hétéroatome choisi parmi l'azote, l'oxygène ou le soufre et éventuellement substitué par alkyle,
      - soit un radical de formule générale : 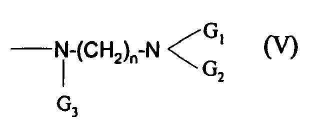 tel que défini précédemment, ou bien
4) les substituants R₁, R₂ et R₄ à R₁₁ et Z₁, Z₂, et Z₄ à Z₁₁ sont tels que définis ci-avant en iv), et R₃ est un substituant :
   - alkyle en C₂ à C₆, alcényle, alcynyle, linéaires ou ramifiés, ces groupes pouvant encore être substitués par un groupe hydroxy, amino, alkylamino en C₁ à C₄, dialkylamino en C₁ à C₃, alkyloxy, ou acyloxy, ou
   - COOG₄ ou CONHG₄, G₄ étant un alkyle contenant 1 à 4 atomes de carbone linéaire ou ramifié, ou
   - -Y-G₅ dans laquelle Y = S, O, et G₅ est un alkyle en C₁ à C₄, un alcényle ou un alcynyle, linéaires ou ramifiés, et dans laquelle si Y = S, G₅ peut être aussi un aryle ou un hétéroaryle, ou
   - un groupement halogène, ou cyano, ou
   - CHG₆G₇, dans laquelle G₆ est un atome d'hydrogène, un groupement méthyle, éthyle, phényle, et G₇ est un atome d'hydrogène, ou un groupe hydroxy, halogène, amino, alkylamino en C₁ à C₄, dialkylamino en C₁ à C₄, acyloxy, *t*-butoxycarbonylamino-éthoxy-éthoxy-acétyloxy, ou alkyloxycarbonyle, ou bien
5) Les substituants R₁, R₂ et R₄ à R₁₁ et Z₁, Z₂, et Z₄ à Z₁₁ sont tels que définis ci-avant en v), et R₃ est un groupement tel que l'on a en position -3 un résidu α-(méthylmercapto)-sarcosyl ou N-méthyl-(D)-alanyl, ou bien
6) Les substituants R₁, R₂ et R₄ à R₁₁ et Z₁, Z₂, et Z₄ à Z₁₁ sont tels que définis ci-avant en vi), et R₃ est un groupement C₁₋₆alkyle, halogénoC₁₋₆alkyle, hydroxyC₁₋₆alkyle, mercaptoC₁₋₆alkyle, amino C₁₋₆alkyle, C₂₋₅alkoxycarbonylamino-(C₁₋₄alkyle), nitroC₁₋₆alkyle, cyanoC₁₋₅alkyle, C₁₋₆alkoxy-(C₁₋₆alkyle), C_{1 -6}alkylthio-(C ₁₋₆alkyle), C₂₋₇alcanoyloxy-(C₁₋₆alkyle), C₂₋₇diazoalcanoyloxy-(C₁₋₆alkyle), carboxy-(C₁₋₆alkyle), C₂₋₇alkoxycarbonyl-(C₁₋₆alkyle), aminocarbonyl-(C ₁₋₄alkyle), aminocarbonyloxy-(C ₁₋₄alkyle), amino-(C ₁₋₄alcanoyloxy)-(C₁₋₄al-kyle), amino-(C₂₋₉alkoxycarbonyl)-(C₁₋₄alkyle), C₂₋₇alkylcarbonyle, C₂₋₇alkoxycarbonyle, C₁₋₆alkylthio, hydroxy-C₁₋₆alkylthio, C₁₋₆alkoxy-(C₁₋₆alkylthio), C₂₋₁₁alcanoyloxy-(C₂₋₄alkylthio), C₂₋₁₁alcanoyloxy-(C₂₋₄alkylsulfinyle), C₂₋₁₁-alcanoyloxy-(C₂₋₄alkylsulfonyle), aminocarbonyloxy-(C₂₋₄alkylthio), C₂₋₁₁amino-alcanoyloxy-(C₂₋₄alkylthio), aminocarbonyloxy-(C₂₋₄alkylsulfinyle), aminocarbonyl-oxy-(C₂₋₄alkylsulfonyle), aminoalcanoyloxy-(C₂₋₄alkylsulfinyle), aminoalcanoyloxy-(C₂₋₄alkylsulfonyle), aminocarbonyle, C₃₋₆alcényle, C₃₋₆alcynyle, halogénoC₃₋₆alcényle, halogénoC₃₋₆alcynyle, hydroxyC₃₋₆alcényle, aryl-(C₁₋₆alkyle), aryl-(C₁₋₆alkyle) hydroxylé, aryl-(C₃₋₆alcényle), aryl-(C₃₋₆alcynyle), aryl-(C₃₋₆alcényle) hydroxylé, aryl-(C₃₋₆alcynyle) hydroxylé, arylthio, hétéroarylthio, aryl-(C₂₋₅alkoxycarbonylamino)-( C₁₋₄alkyle), halogène, cyano, ou un groupe de formule Q-(CH₂-CH₂-O)ₙ-CO-O-CH₂- pour laquelle n est 1, 2, ou 3, et Q est amino, ou bien
7) les substituants R₁, R₂ et R₄ à R₁₁ et Z₁, Z₂, et Z₄ à Z₁₁ sont tels que définis ci-avant en vii), et R₃ est un groupement tel que l'on a en position -3 un acide aminé (D)-MeAla, ou bien
8) les substituants R₁, R₂ et R₄ à R₁₁ et Z₁, Z₂, et Z₄ à Z₁₁ sont tels que définis ci-avant en viii), et R₃ est un groupement tel que l'on a en position -3 un α-acide aminé N-méthylé en position α et ayant la configuration (D).

Les dérivés de cyclosporine de formule générale (IV) peuvent être obtenus par addition sur un polyanion de formule générale (II) d'un agent électrophile. Lorsque les substituants de cet agent peuvent interférer avec la réaction, il est préférable de les protéger préalablement avec des radicaux compatibles et pouvant être mis en place et éliminés sans toucher au reste de la molécule. L'addition peut être suivie le cas échéant des étapes de séparation et de purification du dérivé de cyclosporine de formule (IV) selon les méthodes connues de l'homme du métier.

Les dérivés de cyclosporine de formule générale (IV) dont les substituants sont définis comme en 1), 2), 3), ou 4) pour lesquels R₃ est S-G₅ peuvent être obtenus par addition sur un polyanion de formule générale (II) d'un disulfure de formule générale :

G-S-S-G (VI)

pour lequel G est Alk-R° ou G₅ tels que définis ci-avant en 1), 2), 3), ou 4), les fonctions du polyanion pouvant interférer avec la réaction ayant été le cas échéant préalablement protégées, suivie de l'élimination le cas échéant du/des radicaux protecteurs.

Le disulfure de formule générale (VI) est généralement additionné soit pur soit en solution dans un solvant organique tel qu'un éther aliphatique ou cyclique (par exemple le tétrahydrofuranne, le t-butylméthyléther ou le diméthoxyéthane) ou un hydrocarbure (le toluène par exemple), à une température comprise entre -40 et 0°C. Préférentiellement, l'addition du disulfure est mise en oeuvre à une température comprise entre -25 et -15°C inclus.

Après addition du disulfure de formule générale (VI), le mélange réactionnel est avantageusement porté à une température supérieure à 0°C. Préférentiellement, le mélange réactionnel est maintenu entre 0 et 30°C, et l'évolution de la réaction est suivie selon les méthodes connues de l'homme du métier. Encore plus préférentiellement, le mélange réactionnel est maintenu à une température comprise entre 15 et 25°C inclus.

Le disulfure de formule générale (VI) peut être obtenu à partir de deux équivalents d'un composé de formule générale G-SH pour lequel G est Alk-R° ou G₅ tels que définis précédemment en 1), 2), 3), ou 4). On opère en milieu oxydant, dans un solvant organique (par exemple dans l'oxyde de diéthyle ou dans le dichlorométhane) ou dans un alcool (par exemple le méthanol ou l'éthanol), et en présence d'un hydroxyde alcalin. Notamment, le milieu oxydant est obtenu par passage d'oxygène ou par adjonction de diiode dans un solvant organique, par exemple l'oxyde de diéthyle. De façon préférée, l'hydroxyde alcalin est l'hydroxyde de sodium.

Lorsque les substituants du radical G peuvent interférer avec la réaction, il est préférable de les protéger préalablement par des radicaux compatibles et pouvant être mis en place et éliminés sans toucher au reste de la molécule. De plus les radicaux hydroxy présents sur la cyclosporine peuvent être éventuellement protégés par tout groupement qui n'interfère pas avec la réaction.

A titre d'exemple, les groupements protecteurs peuvent être choisis parmi les radicaux décrits par T.W. GREENE, Protective Groups in Organic Synthesis, J. Wiley-Inter-science Publication (1991) ou par Mc Omie, Protective Groups in Organic Chemistry, Plenum Press (1973).

Dans la formule générale (V), lorsque G₁ et/ou G₂ représentent un substituant hétérocyclyle, celui-ci peut être avantageusement choisi parmi pyridyle, tétrahydropyridyle, pipéridyle, imidazolyle, oxazolyle, thiazolyle.

Lorsque G₁ et G₂ forment un hétérocycle avec l'atome d'azote auquel ils sont rattachés, le radical hétérocyclyle peut être choisi à titre d'exemple parmi azétidinyle, pipéridyle, pipérazinyle, N-méthyl pipérazinyle, N-phényl pipérazinyle, N-benzyl pipérazinyle, pyridyle, imidazolyle, morpholino, thiomorpholino, tétrahydropyridyle, méthyl tétrahydropyridyle (par exemple 4-méthyl tétrahydropyridyle), phényl tétrahydropyridyle (par exemple 4-phényl tétrahydropyridyle).

La réaction de thioalkylation peut être suivie le cas échéant des étapes de séparation et de purification du dérivé de cyclosporine de formule générale (IV) selon les méthodes connues de l'homme du métier. Notamment, on peut opérer par les méthodes physiques telles que la cristallisation ou la chromatographie.

Les dérivés de formule générale (IV) pour laquelle les substituants R₁ à R₁₁ et Z₁ à

Z₁₁ sont tels que définis en 4) à l'exception de R₃ représentant S-G₅, peuvent être obtenus à partir du polyanion selon l'invention par les méthodes décrites dans la demande de brevet WO 97/04005 ou par toute autre méthode équivalente.

Les dérivés de formule générale (IV) pour laquelle les substituants R₁ à R₁₁ et Z₁ à Z₁₁ sont tels que définis en 5) peuvent être obtenus à partir du polyanion selon l'invention par analogie avec les méthodes décrites dans la demande de brevet EP 194972.

Les dérivés de formule générale (IV) pour laquelle les substituants R₁ à R₁₁ et Z₁ à Z₁₁ sont tels que définis en 6) peuvent être obtenus à partir du polyanion selon l'invention par les méthodes décrites dans la demande de brevet EP 194 972 ou par toute autre méthode équivalente.

Les dérivés de formule générale (IV) pour laquelle les substituants R₁ à R₁₁ et Z₁ à Z₁₁ sont tels que définis en 7) peuvent être obtenus à partir du polyanion selon l'invention par analogie avec les méthodes décrites dans la demande de brevet EP 194 972.

Les dérivés de formule générale (IV) pour laquelle les substituants R₁ à R₁₁ et Z₁ à Z₁₁ sont tels que définis en 8) peuvent être obtenus à partir du polyanion selon l'invention par analogie avec les méthodes décrites dans la demande de brevet EP 194 972.

Il est entendu que les dérivés de cyclosporine obtenus peuvent être éventuellement transformés en sels lorsqu'ils existent.

Les dérivés de cyclosporine tels que définis en 1), 2), 3), 4), et 7) sont utiles pour le traitement et/ou la prophylaxie des infections à rétrovirus, et plus particulièrement du SIDA (syndrome d'immuno-déficience acquise) et de syndromes associés [ARC (AIDS related complex)]. Ils présentent l'avantage d'être très faiblement immunosuppresseurs.

Les dérivés de cyclosporine tels que définis en 5) présentent une activité immunosuppressive, et sont donc utiles pour le traitement de diverses maladies inflammatoires chroniques et des maladies auto-immunes.

Les dérivés de cyclosporine tels que définis en 6) et en 8) possèdent :
- soit une activité immunosuppressive, et ils sont donc utiles notamment pour le traitement et/ou la prophylaxie des maladies auto-immunes ou pour prévenir le rejet d'organes transplantés,
- soit une activité anti-inflammatoire, et ils sont donc notamment utiles pour le traitement d'inflammations comme par exemple l'arthrite et les maladies rhumatismales,
- soit une activité anti-parasitaire, et ils sont par exemple utiles pour le traitement de la schistosomiasis, la filariasis, la leishmaniasis, la coccidioidomycosis, ou de la malaria.

Les exemples suivants, donnés à titre non-limitatif, illustrent la présente invention.

Exemple 1 : préparation de la [(R)-2-(N,N-diméthylamino)éthylthio-Sar]³ [4'-hydroxy-MeLeu]⁴ cyclosporine A. Dans un réacteur sous atmosphère d'azote, maintenu à -20°C et contenant une solution de 36,8 g de sel de lithium de l'hexaméthyldisilazane dans 104 cm³ de t-butylméthyléther, on introduit 68 cm³ de toluène et 40 cm³ de t-butylméthyléther. Sous agitation, on ajoute une portion de 9,6 g de [4'-hydroxy-MeLeu]⁴ cyclosporine A. Le mélange est agité pendant 15 minutes à une température voisine de -20°C, puis 8,3 g de chlorure de césium sont ajoutés en une portion. Le mélange réactionnel est agité durant 30 minutes, puis 25,5 g de disulfure de di-[2-(N,N-diméthylamino)éthyle] sont ajoutés en 30 minutes. Le mélange réactionnel est alors porté à une température voisine de 25°C en approximativement 1 heure et 30 minutes. Après 2 heures d'agitation, on refroidit le mélange réactionnel à une température voisine de -10°C et on coule 14,4 cm³ d'acide acétique. Enfm, on ajoute 200 ml d'eau sans maintenir la température puis la phase organique est séparée après décantation.
L'analyse CLHP (Chromatographie Liquide Haute Performance) par étalonnage externe de la phase organique indique que le mélange réactionnel contient 3,95 g [(R)-2-(N,N-diméthylamino)éthylthio-Sar]³ [4'-hydroxy-MeLeu]⁴ cyclosporine A, ce qui correspond à un rendement molaire de 38%.

L'isolement de la [(R)-2-(N,N-diméthylamino)éthylthio-Sar]³ [4'-hydroxy-MeLeu]⁴ cyclosporine A est réalisé de la façon suivante :
Après ajout de 100 cm³ d'eau distillée à la phase organique, le mélange est refroidi à 5°C. En approximativement 30 minutes et sous agitation, le pH de la phase aqueuse est amené à 6,5 par un ajout goutte à goutte d'acide méthanesulfonique (environ 21 cm³). A la phase organique décantée, on ajoute ensuite 20 g d'alumine et le mélange est agité pendant 10 minutes. Le mélange est filtré, et le solide récupéré est rincé par du t-butylméthyléther. Les phases organiques sont ensuite réunies et on additionne 200 ml d'eau distillée. Sous agitation, le pH est alors ajusté à 2 par ajout d'acide méthanesulfonique. Le mélange est agité durant 1 heure, et 50 cm³ d'acétonitrile sont additionnés. Enfm, la phase organique est décantée puis écartée.
La phase aqueuse est concentée sous pression réduite (2,7 kPa) à une température voisine de 30°C jusqu'à l'élimination complète des solvants organiques. Puis, sous agitation, le pH de la phase aqueuse est amené à 9 par ajout d'ammoniaque à 20%. La [(R)-2-(N,N-diméthylamino)éthylthio-Sar]³ [4'-hydroxy-MeLeu]⁴ cyclosporine A brute précipite alors dans la phase aqueuse. Après isolement par filtration du précipité, puis séchage à 40°C pendant environ 12 heures sous pression réduite (2,7 kPa), on obtient 6 g de [(R)-2-(N,N-diméthylamino)éthylthio-Sar]³ [4'-hydroxy-MeLeu]⁴ cyclosporine A brute.
Une portion de 1 g de [(R)-2-(N,N-diméthylamino)éthylthio-Sar]³ [4'-hydroxy-MeLeu]⁴ cyclosporine A brute est purifiée par chromatographie sur une colonne de silice (0,63-0,20 mm; éluant : acétonitrile-méthanol/ammoniac (20 % aq.) 85:15:1 en volume). Les fractions contenant le produit attendu sont concentrées à sec sous pression réduite (2,7 kPa) pour donner 0,4 g de [(R)-2-(N,N-diméthylamino)éthylthio-Sar]³ [4'-hydroxy-MeLeu]⁴ cyclosporine A sous la forme d'un solide blanc.

Spectre de R.M.N. ¹H (400 MHz, CDCl₃, δ en ppm) : 1,23 (d, J = 7 Hz, 3H : CH₃ 8β); 1,33 (d, J = 7,5 Hz, 3H : CH₃ 7β); 1,60 (d, J = 5 Hz, 3H : CH₃ 1η); 1,68 et 2,36 (2 dd, J = 15 et 6,5 Hz, 1H chacun : CH₂ 4β); 2,23 (s large, 6H : N(CH₃)₂ du 2-diméthylaminoéthylthio en 3α); 2,40 (mt, 1H : CH 5β); de 2,50 à 2,85 (mt, 4H : SCH₂CH₂N du 2-diméthylaminoéthylthio en 3α); 2,68 - 3,09 - 3,16 - 3,22 - 3,42 et 3,47 (6 s, respectivement 6H - 3H - 3H - 3H - 3H et 3H : 7 NCH₃); 3,63 (d, J = 6 Hz, 1H : OH en 1β); 3,72 (mt, 1H : CH 1β); 4,52 (mt, 1H : CH 7α); 4,61 (t, J = 9 Hz, 1H : CH 5α); 4,81 (mt, 1H : CH 8α); 4,95 (dd, J = 9 et 6 Hz, 1H : CH α d'une leucine); de 5,00 à 5,10 (mt, 2H : CH 2α et CH α d'une leucine); 5,10 (d, J = 11 Hz, 1H : CH 11α); de 5,20 à 5,35 (mt, 2H : CH=CH); 5,40 (t, J = 6,5 Hz, 1H : CH 4α); 5,47 (d, J = 6 Hz, 1H : CH 1α); 5,68 (dd, J = 10,5 et 4 Hz, 1H : CH α d'une leucine); 5,96 (s, 1H : CH 3α); 7,12 (d, J = 8 Hz, 1H : CONH en 8); 7,46 (d, J = 9 Hz, 1H : CONH en 5); 7,60 (d, J = 7,5 Hz, 1H : CONH en 7); 7,92 (d, J = 9,5 Hz, 1H : CONH en 2).

**Exemple 2 :** préparation du sel de méthanesulfonate de la [(R)-2-(N,N-diméthylamino)éthylthio-Sar]³ cyclosporine A. Dans un réacteur sous atmosphère d'azote maintenu à -20°C et contenant une solution de 20,8 g de sel de lithium de l'hexaméthyldisilazane dans 58 cm³ de t-butylméthyléther, on introduit 38 cm³ de toluène et 25 cm³ de t-butylméthyléther. Sous agitation, on ajoute en une portion 5,1 g de cyclosporine A puis 15 cm³ de t-butylméthyléther. Le mélange est agité pendant 15 minutes à une température voisine de -20°C puis 4,4 g de chlorure de césium sont ajoutés en une portion. Le mélange réactionnel est agité pendant 30 minutes, puis 13,9 g de disulfure de di-[2-(N,N-diméthylamino)éthyle] sont ajoutés en 10 minutes. Le mélange réactionnel est porté à une température voisine de 25°C en approximativement 1 heure et 30 minutes. Après 1 heure d'agitation, on refroidit le mélange réactionnel à une température d'environ -10°C et on coule 10 cm³ d'acide acétique. Enfin, on ajoute 75 cm³ d'eau sans maintenir la température, puis la phase organique est décantée.
L'analyse CLHP (Chromatographie Liquide Haute Performance) par étalonnage externe de la phase organique indique que le mélange réactionnel contient 2,9 g [(R)-2-(N,N-diméthylamino)éthylthio-Sar]³ cyclosporine A, ce qui correspond à un rendement molaire de 53 %.

L'isolement du sel de méthanesulfonate de la [(R)-2-(N,N-diméthylamino)éthylthio-Sar]³ cyclosporine A est réalisé de la façon suivante :
La phase organique obtenue précédement est concentrée à sec sous pression réduite (2,7 kPa), à une température voisine de 35°C. Le résidu huileux orange obtenu (16,13 g) est traité par 240 cm³ d'éther de diéthyle, 1,3 litre d'eau distillée et 40 cm³ d'acide chlorhydrique 5N. La phase organique est décantée et écartée.
La phase aqueuse est extraite par 250 cm³ d'éther de diéthyle, puis elle est neutralisée par ajout de bicarbonate de sodium solide jusqu'à saturation. La dite phase aqueuse neutralisée est alors extraite par 250 cm³ d'éther de diéthyle. La phase organique est décantée et séparée, et la phase aqueuse est à nouveau extraite par 250 cm³ d'éther de diéthyle.
Les phases organiques réunies sont lavées par 180 cm³ au total d'une solution saturée en chlorure de sodium, séchées sur sulfate de sodium, puis filtrées. Le filtrat est concentré à sec sous pression réduite (2,7 kPa) à une température voisine de 30°C pour conduire à 4,5 g de [(R)-2-(N,N-diméthylamino)éthylthio-Sar]³ cyclosporine A brute sous la forme d'une meringue crème. Une portion de 1 g de la [(R)-2-(N,N-diméthylamino)éthylthio-Sar]³ cyclosporine A brute est purifiée par chromatographie sur colonne d'alumine neutre (éluant : cyclohexane/acétate d'éthyle, 1:4 en volume). Les fractions contenant le produit attendu sont réunies et concentrées à sec sous pression réduite (2,7 kPa) à une température voisine de 35°C pour donner 0,4 g d'une meringue blanche. La [(R)-2-(N,N-diméthylamino)éthylthio-Sar]³ cyclosporine A obtenue est rechromatographié sur colonne d'alumine neutre (éluant : cyclohexane/acétate d'éthyle, 1:4 en volume). Les fractions contenant le produit attendu sont réunies et concentrées à sec sous pression réduite (2,7 kPa) à une température voisine de 35°C pour donner 0,1 g d'une meringue blanche.
La dite meringue blanche est mise en solution dans 0,5 cm³ d'éther de diéthyle. A cette solution sont ajoutés 0,4 cm³ d'une solution 0,2 N d'acide méthanesulfonique dans l'éther de diéthyle. Après 1 heure d'agitation à une température voisine de 20°C, le mélange est filtré. Le solide est rincé par 2 fois 0,1 cm³ d'éther de diéthyle. Après séchage du solide à poids constant sous pression réduite (2,7 kPa), à une température voisine de 30°C durant 24 heures, on obtient 0,06 g de sel de méthanesulfonate de la [(R)-2-(N,N-diméthylamino)éthylthio-Sar]³ cyclosporine A sous la forme d'un solide blanc.

Spectre de R.M.N. ¹H (400 MHz, (CD₃)₂SO d6, δ en ppm) : 1,21 (d, J = 7,5 Hz, 3H : CH₃ 8β); 1,29 (d, J = 7,5 Hz, 3H : CH₃ 7β); 1,69 (d, J = 6,5 Hz, 3H : CH₃ 1η); 1,99 (mt, 1H : CH 5β); 2,35 (s, 3H : CH₃ du méthanesulfonate); de 2,45 à 2,70 (mt, 2H : SCH₂ du 2-diméthyl aminoéthylthio en 3α); 2,64 - 2,80 - 2,86 - 2,93 - 2,99 et 3,17 (6 s, respectivement 3H - 6H - 9H - 3H - 3H et 3H : 7 NCH₃ et NCH₃ du 2-diméthyl aminoéthylthio en 3α); de 3,25 à 3,40 (mt, 2H : CH₂N du 2-diméthylaminoéthylthio en 3α); 3,99 (mt, 1H : CH 1β ; 4,15 (mt, 1H : CH 7α); 4,26 (t, J = 9 Hz, 1H : CH 5α); 4,42 (s large, 1H : OH en 1β); 4,79 (mt, 1H : CH 8α); 4,89 (mt, 1H : CH 2α); de 5,00 à 5,15 (mt, 1H : CH α d'une leucine); 5,11 (d, J =11 Hz, 1 H : CH 11α); 5,23 (mt, 2H : CH 1α et CH α d'une leucine); 5,33 (dd, J = 10 et 5 Hz, 1H : CH α d'une leucine); de 5,30 à 5,50 et 5,62 (2 mts, 1H chacun : CH=CH); 5,48 (dd, J = 11 et 5 Hz, 1H : CH α d'une leucine); 6,87 (s, 1H : CH 3α); 7,64 (d, J = 7,5 Hz, 1H : CONH en 7); 8,24 (d, J = 9,5 Hz, 1H : CONH en 2); 8,28 (d, J = 8 Hz, 1H : CONH en 8); 8,68 (d, J = 9 Hz, 1H : CONH en 5); 9,28 (mf, 1H : SO₃H du méthanesulfonate).

### Exemple 3 : préparation de la [(R)-méthylthio-Sar]³ [4'-hydroxy-MeLeu]⁴ cyclosporine A

Dans un réacteur sous atmosphère d'azote, maintenu à -20°C et contenant 19 g de sel de lithium de l'hexaméthyldisilazane, on introduit 85 cm³ de tétrahydrofuranne et 22,5 cm³ de toluène. Sous agitation, on ajoute en une portion 4,8 g de cyclosporine A puis 10 cm³ de tétrahydrofuranne. Le mélange est agité pendant 15 minutes à une température voisine de -20°C puis 4,46 g de chlorure de césium sont ajoutés en une portion. Le mélange réactionnel est agité durant 15 minutes, puis 5,8 g de disulfure de diméthyle sont ajoutés en approximativement 15 minutes. Le mélange réactionnel est porté à une température voisine de 25°C en approximativement 2 heures 30 minutes. Après 4 heures d'agitation, on refroidit le mélange réactionnel à une température voisine de -10°C et on coule 10 cm³ d'acide acétique. On ajoute 75 cm³ d'eau sans maintenir la température puis la phase organique est décantée. L'analyse CLHP (Chromatographie Liquide Haute Performance) par étalonnage externe de la phase organique indique que le mélange réactionnel contient 2,3 g [(R)-méthylthio-Sar]³ [4'-hydroxy-MeLeu]⁴ cyclosporine A, ce qui correspond à un rendement molaire de 46%.

L'isolement de la [(R)-méthylthio-Sar]³ [4'-hydroxy-MeLeu]⁴ cyclosporine A peut être réalisé de la façon suivante :
A la phase organique obtenue précédement, on ajoute 500 cm³ d'eau. Sous agitation, on coule 40 cm³ d'acide chlorhydrique 5 N en environ 15 minutes. Après 15 minutes d'agitation, la phase organique est décantée puis concentrée à sec sous pression réduite (2,7 kPa) à une température voisine de 35°C. On obtient alors 6,3 g de [(R)-méthylthio-Sar]³ [4'-hydroxy-MeLeu]⁴ cyclosporine A brute sous la forme d'une meringue jaunâtre.
Une portion de 0,1 g de [(R)-méthylthio-Sar]³ [4'-hydroxy-MeLeu]⁴ cyclosporine A brute est purifiée par chromatographie sur couche mince de silice préparative (éluant : dichlorométhane/acétonitrile/propanol-2, 65/25/10 en volume). La silice contenant le produit attendu est prélevée et agitée avec 5 cm³ de dichlorométhane. Après filtration et évaporation sous pression réduite (2,7 kPa) à une température voisine de 30°C, on obtient 0,035 g de [(R)-méthylthio-Sar]³ [4'-hydroxy-MeLeu]⁴ cyclosporine A sous la forme d'un film incolore.

Spectre de R.M.N. ¹H (400 MHz, CDCl₃, δ en ppm) : 1,27 (d, J = 7 Hz, 3H : CH₃ 8β); 1,37 (d, J = 7,5 Hz, 3H : CH₃ 7β); 1,64 (d, J = 5 Hz, 3H : CH₃ en 1η); de 1.65 à 1.80 et 2,41 (respectivement mt et dd, J = 15 et 6,5 Hz, 1H chacun : CH₂ 4β); 2,17 (s, 3H : SCH₃); 2,47 (mt, 1H : CH 5β); 2,71 - 3,13 - 3,18 - 3,27 - 3,46 et 3,52 (6 s, respectivement 6H - 3H - 3H - 3H - 3H et 3H : 7 NCH₃); 3,70 (d, J = 6,5 Hz, 1H : OH en 1β); 3,78 (mt, 1H : CH 1β); 4,56 (mt, 1H : CH 7α); 4,67 (t, J = 9 Hz, 1H : CH 5α); 4,86 (mt, 1H : CH 8α); 5,00 (dd, J = 9 et 6 Hz, 1H : CH α d'une leucine); de 5,05 à 5,15 (mt, 2H : CH 2α et CH α d'une leucine); 5,15 (d, J = 11 Hz, 1 H : CH 11α); de 5,25 à 5,40 (mt, 2H : CH=CH); 5,45 (t, J = 6,5 Hz, 1H : CH 4α); 5,52 (d, J = 6 Hz, 1H : CH 1α); 5,72 (dd, J = 10,5 et 4 Hz, 1H : CH α d'une leucine); 5,75 (s, 1H : CH 3α); 7,16 (d, J = 8 Hz, 1H : CONH en 8); 7,52 (d, J = 9 Hz, 1H : CONH en 5); 7,65 (d, J = 7,5 Hz, 1H : CONH en 7); 7,94 (d, J = 9,5 Hz, 1H : CONH en 2).

### Exemple 4

En opérant de manière analogue à la méthode décrite dans les exemples précédents, on prépare les produits suivants :
[(R)-2-aminoéthylthio-Sar]³-cyclosporine A ;
[(R)-2-(N-méthylamino)éthylthio-Sar]³-cyclosporine A ;
[(R)-2-(N-éthylamino)éthylthio-Sar]³-cyclosporine A ;
[(R)-2-(N-*i*.propylamino)éthylthio-Sar]³-cyclosporine A ;
[(R)-2-(N-*t*.butylamino)éthylthio-Sar]³-cyclosporine A ;
[(R)-2-(N-phénylamino)éthylthio-Sar]³-cyclosporine A ;
[(R)-2-(N-benzylamino)éthylthio-Sar]³-cyclosporine A ;
[(R)-2-(N-méthyl-N-éthylamino)éthylthio-Sar]³-cyclosporine A ;
[(R)-2-(N-méthyl-N-allylamino)éthylthio-Sar]³-cyclosporine A ;
[(R)-2-(N-méthyl-N-phénylamino)éthylthio-Sar]³-cyclosporine A ;
[(R)-2-(N,N-di-*i*.propylamino)éthylthio-Sar]³-cyclosporine A ;
[(R)-2-(N,N-diallylamino)éthylthio-Sar]³-cyclosporine A ;
[(R)-3-aminopropylthio-Sar]³-cyclosporine A ;
[(R)-3-(N-méthylamino)propylthio-Sar]³-cyclosporine A ;
[(R)-3-(N-éthylamino)propylthio-Sar]³-cyclosporine A ;
[(R)-3-(N-*i*.propylamino)propylthio-Sar]³-cyclosporine A ;
[(R)-3-(N-*t*.butylamino)propylthio-Sar]³-cyclosporine A ;
[(R)-3-(N-phénylamino)propylthio-Sar]³-cyclosporine A ;
[(R)-3-(N-benzylamino)propylthio-Sar]³-cyclosporine A ;
[(R)-3-(N-méthyl-N-éthylamino)propylthio-Sar]³-cyclosporine A ;
[(R)-3-(N-méthyl-N-*i*.propyllamino)propylthio-Sar]³-cyclosporine A ;
[(R)-3-(N-méthyl-N-*t*.butylamino)propylthio-Sar]³-cyclosporine A ;
[(R)-3-(N-méthyl-N-allylamino)propylthio-Sar]³-cyclosporine A ;
[(R)-3-(N-méthyl-N-phénylamino)propylthio-Sar]³-cyclosporine A ;
[(R)-3-(N-méthyl-N-benzylamino)propylthio-Sar]³-cyclosporine A ;
[(R)-3-(N,N-diéthylamino)propylthio-Sar]³-cyclosporine A ;
[(R)-3-(N,N-di-i.propylamino)propylthio-Sar]³-cyclosporine A ;
[(R)-3-(N,N-diallylamino)propylthio-Sar]³-cyclosporine A ;
[(R)-3-(1-pipéridyl)propylthio-Sar]³-cyclosporine A ;
[(R)-4-aminobutylthio-Sar]³-cyclosporine A ;
[(R)-4-(N-méthylamino)butylthio-Sar]³-cyclosporine A ;
[(R)-4-(N-éthylamino)butylthio-Sar]³-cyclosporine A ;
[(R)-4-(N-*i*.propylamino)butylthio-Sar]³-cyclosporine A ;
[(R)-4-(N-*t*.butylamino)butylthio-Sar]³-cyclosporine A ;
[(R)-4-(N-phénylamino)butylthio-Sar]³-cyclosporine A ;
[(R)-4-(N-benzylamino)butylthio-Sar]³-cyclosporine A ;
[(R)-4-(N-méthyl-N-éthylamino)butylthio-Sar]³-cyclosporine A ;
[(R)-4-(N-méthyl-N-*i*.propylamino)butylthio-Sar]³-cyclosporine A ;
[(R)-4-(N-méthyl-N-*t*.butylamino)butylthio-Sar]³-cyclosporine A ;
[(R)-4-(N-méthyl-N-allylamino)butylthio-Sar]³-cyclosporine A ;
[(R)-4-(N-méthyl-N-phénylamino)butylthio-Sar]³-cyclosporine A ;
[(R)-4-(N-méthyl-N-benzylamino)butylthio-Sar]³-cyclosporine A ;
[(R)-4-(N,N-diméthylamino)butylthio-Sar]³-cyclosporine A ;
[(R)-4-(N,N-diéthylamino)butylthio-Sar]³-cyclosporine A ;
[(R)-4-(N,N-di-*i*.propylamino)butylthio-Sar]³-cyclosporine A ;
[(R)-4-(N,N-diallylamino)butylthio-Sar]³-cyclosporine A ;
[(R)-4-(1-pipéridyl)butylthio-Sar]³-cyclosporine A ;
[(R)-2-amino-2-méthylpropylthio-Sar]³-cyclosporine A ;
[(R)-2-(N,N-diméthylamino)-2-méthylpropylthio-Sar]³-cyclosporine A ;
[(R)-2-(N,N-diéthylamino)-2-méthylpropylthio-Sar]³-cyclosporine A ;
[(R)-2-(1-pipéridyl)-2-méthylpropylthio-Sar]³-cyclosporine A ;
[(R)-3-amino-3-méthylbutylthio-Sar]³-cyclosporine A ;
[(R)-3-(N,N-diméthylamino)-3-méthylbutylthio-Sar]³-cyclosporine A ;
[(R)-3-(N,N-diéthylamino)-3-méthylbutylthio-Sar]³-cyclosporine A ;
[(R)-3-(1-pipéridyl)-3-méthylbutylthio-Sar]³-cyclosporine A ;
[(R)-2-(1-morpholino)éthylthio-Sar]³-cyclosporine A ;
[(R)-2-(1-azétidino)éthylthio-Sar]³-cyclosporine A ;
{(R)-2-[1-(4-méthylpipérazino)]éthylthio-Sar}³-cyclosporine A ;
{(R)-2-[1-(4-phénylpipérazino)]éthylthio-Sar}³-cyclosporine A ;
{(R)-2-[1-(4-benzylpipérazino)]éthylthio-Sar}³-cyclosporine A ;
{(R)-2-[1-(4-méthyl-1,2,3,6-tétrahydropyridyl)]éthylthio-Sar}³-cyclosporine A ;
{(R)-2-[1-(4-phényl-1,2,3,6-tétrahydropyridyl)]éthylthio-Sar}³-cyclosporine A ;
[(R)-3-(1-morpholino)propylthio-Sar]³-cyclosporine A ;
[(R)-3-(1-azétidino)propylthio-Sar]³-cyclosporine A ;
{(R)-3-[1-(4-méthylpipérazino)]propylthio-Sar}³-cyclosporine A ;
{(R)-3-[1-(4-phénylpipérazino)]propylthio-Sar}³-cyclosporine A ;
{(R)-3-[1-(4-benzylpipérazino)]propylthio-Sar}³-cyclosporine A ;
{(R)-3-[1-(4-méthyl-1,2,3,6-tétrahydropyridyl)]propylthio-Sar}³-cyclosporine A ;
{(R)-3-[1-(4-méthyl-1,2,3,6-tétrahydropyridyl)]propylthio-Sar}³-cyclosporine A.
[(R)-2-aminoéthylthio-Sar]³-[4'-hydroxy-MeLeu]⁴-cyclosporine A ;
[(R)-2-(N-méthylamino)éthylthio-Sar]³-[4'-hydroxy-MeLeu]⁴-cyclosporine A ;
[(R)-2-(N-éthylamino)éthylthio-Sar]³-[4'-hydroxy-MeLeu]⁴-cyclosporine A ;
[(R)-2-(N-*iso*-propylamino)éthylthio-Sar]³-[4'-hydroxy-MeLeu]⁴-cyclosporine A ;
[(R)-2-(N-*t*-butylamino)éthylthio-Sar]³-[4'-hydroxy-MeLeu]⁴-cyclosporine A ;
[(R)-2-(N-phénylamino)éthylthio-Sar]³-[4'-hydroxy-MeLeu]⁴-cyclosporine A ;
[(R)-2-(N-benzylamino)éthylthio-Sar]³-[4'-hydroxy-MeLeu]⁴-cyclosporine A ;
[(R)-2-(N-méthyl-N-éthylamino)éthylthio-Sar]³-[4'-hydroxy-MeLeu]⁴-cyclosporine A;
[(R)-2-(N-méthyl-N-iso-propylamino)éthylthio-Sar]³-[4'-hydroxy-MeLeu]⁴-cyclosporine A ;
[(R)-2-(N-méthyl-N-tert-butylamino)éthylthio-Sar]³-[4'-hydroxy-MeLeu]⁴-cyclosporine A ;
[(R)-2-(N-méthyl-N-allylamino)éthylthio-Sar]³-[4'-hydroxy-MeLeu]⁴-cyclosporine A;
[(R)-2-(N-méthyl-N-phénylamino)éthylthio-Sar]³-[4'-hydroxy-MeLeu]⁴-cyclosporine A ;
[(R)-2-(N-méthyl-N-benzylamino)éthylthio-Sar]³-[4'-hydroxy-MeLeu]⁴-cyclosporine A;
[(R)-2-(N,N-diéthylamino)éthylthio-Sar]³-[4'-hydroxy-MeLeu]⁴-cyclosporine A ;
[(R)-2-(N,N-di-iso-propylamino)éthylthio-Sar]³-[4'-hydroxy-MeLeu]⁴-cyclosporine A ;
[(R)-2-(N,N-diallylamino)éthylthio-Sar]³-[4'-hydroxy-MeLeu]⁴-cyclosporine A ;
[(R)-2-(1-pipéridyl)éthylthio-Sar]³-[4'-hydroxy-MeLeu]⁴-cyclosporine A ;
[(R)-3-aminopropylthio-Sar]³-[4'-hydroxy-MeLeu]⁴-cyclosporine A ;
[(R)-3-(N-méthylamino)propylthio-Sar]³-[4'-hydroxy-MeLeu]⁴-cyclosporine A ;
[(R)-3-(N-éthylamino)propylthio-Sar]³-[4'-hydroxy-MeLeu]⁴-cyclosporine A ;
[(R)-3-(N-iso-propylamino)propylthio-Sar]³-[4'-hydroxy-MeLeu]⁴-cyclosporine A ;
[(R)-3-(N-tert-butylamino)propylthio-Sar]³-[4'-hydroxy-MeLeu]⁴-cyclosporine A ;
[(R)-3-(N-phénylamino)propylthio-Sar]³-[4'-hydroxy-MeLeu]⁴-cyclosporine A ;
[(R)-3-(N-benzylamino)propylthio-Sar]³-[4'-hydroxy-MeLeu]⁴-cyclosporine A ;
[(R)-3-(N-méthyl-N-éthylamino)propylthio-Sar]³-[4'-hydroxy-MeLeu]⁴-cyclosporine A ;
[(R)-3-(N-méthyl-N-*i*-propylamino)propylthio-Sar]³-[4'-hydroxy-MeLeu]⁴-cyclosporine A ;
[(R)-3-(N-méthyl-N-t-butylamino)propylthio-Sar]³-[4'-hydroxy-MeLeu]⁴-cyclosporine A ;
[(R)-3-(N-méthyl-N-allylamino)propylthio-Sar]³-[4'-hydroxy-MeLeu]⁴-cyclosporine A;
[(R)-3-(N-méthyl-N-phénylamino)propylthio-Sar]³-[4'-hydroxy-MeLeu]⁴-cyclosporine A ;
[(R)-3-(N-méthyl-N-benzylamino)propylthio-Sar]³-[4'-hydroxy-MeLeu]⁴-cyclosporine A ;
[(R)-2-(N,N-diméthylamino)éthylthio-Sar]³-[4'-hydroxy-MeLeu]⁴-cyclosporine A ;
[(R)-3-(N,N-diéthylamino)propylthio-Sar]³-[4'-hydroxy-MeLeu]⁴-cyclosporine A ;
[(R)-3-(N,N-di-*i*-propylamino)propylthio-Sar]³-[4'-hydroxy-MeLeu]⁴-cyclosporine A;
[(R)-3-(N,N-diallyalamino)propylthio-Sar]³-[4'-hydroxy MeLeu]⁴-cyclosporine A ;
[(R)-3-(1-pipéridyl)propylthio-Sar]³-[4'-hydroxy-MeLeu]⁴-cyclosporine A ;
[(R)-4-aminobutylthio-Sar]³-[4'-hydroxy-MeLeu]⁴-cyclosporine A ;
[(R)-4-(N-méthylamino)butylthio-Sar]³-[4'-hydroxy-MeLeu]⁴-cyclosporine A ;
[(R)-4-(N-éthylamino)butylthio-Sar]³-[4'-hydroxy-MeLeu]⁴-cyclosporine A ;
[(R)-4-(N-*i*-propylamino)butylthio-Sar]³-[4'-hydroxy-MeLeu]⁴-cyclosporine A ;
[(R)-4-(N-*t*-butylamino)butylthio-Sar]³-[4'-hydroxy-MeLeu]⁴-cyclosporine A ;
[(R)-4-(N-phénylamino)butylthio-Sar]³-[4'-hydroxy-MeLeu]⁴-cyclosporine A ;
[(R)-4-(N-benzylamino)butylthio-Sar]³-[4'-hydroxy-MeLeu]⁴-cyclosporine A ;
[(R)-4-(N-méthyl-N-éthylamino)butylthio-Sar]³-[4'-hydroxy-MeLeu]⁴-cyclosporine A;
[(R)-4-(N-méthyl-N-*i*-propylamino)butylthio-Sar]³-[4'-hydroxy-MeLeu]⁴-cyclosporine A ;
[(R)-4-(N-méthyl-N-*t*-butylamino)butylthio-Sar]³-[4'-hydroxy MeLeu]⁴-cyclosporine A;
[(R)-4-(N-méthyl-N-allylamino)butylthio-Sar]³-[4'-hydroxy-MeLeu]⁴-cyclosporine A;
[(R)-4-(N-méthyl-N-phénylamino)butylthio-Sar]³-[4'-hydroxy-MeLeu]⁴-cyclosporine A;
[(R)-4-(N-méthyl-N-benzylamino)butylthio-Sar]³-[4'-hydroxy-MeLeu]⁴-cyclosporine A;
[(R)-4-(N,N-diméthylamino)butylthio-Sar]³-[4'-hydroxy-MeLeu]⁴-cyclosporine A ;
[(R)-4-(N,N-diéthylamino)butylthio-Sar]³-[4'-hydroxy-MeLeu]⁴-cyclosporine A ;
[(R)-4-(N,N-di-*i*-propylamino)butylthio-Sar]³-[4'-hydroxy-MeLeu]⁴-cyclosporine A;
[(R)-4-(N,N-diallyalamino)butylthio-Sar]³-[4'-hydroxy-MeLeu]⁴-cyclosporine A ;
[(R)-4-(1-pipéridyl)butylthio-Sar]³-[4'-hydroxy-MeLeu]⁴-cyclosporine A ;
[(R)-2-amino-2-méthylpropylthio-Sar]³-[4'-hydroxy-MeLeu]⁴-cyclosporine A ;
[(R)-2-(N,N-diméthylamino)-2-méthylpropylthio-Sar]³-[4'-hydroxy-MeLeu]⁴-cyclosporine A ;
[(R)-2-(N,N-diéthylamino)-2-méthylpropylthio-Sar]³-[4'-hydroxy-MeLeu] ⁴-cyclosporine A ;
[(R)-2-(1-pipéridyl)-2-méthylpropylthio-Sar]³-[4'-hydroxy-MeLeu]⁴-cyclosporine A ;
[(R)-3-amino-3-méthylbutylthio-Sar]³-[4'-hydroxy-MeLeu]⁴-cyclosporine A ;
[(R)-3-(N,N-diméthylamino)-3-méthylbutylthio-Sar]³-[4'-hydroxy-MeLeu]⁴-cyclosporine A ;
[(R)-3-(N,N-diéthylamino)-3-méthylbutylthio-Sar]³-[4'-hydroxy-MeLeu]⁴-cyclosporine A ;
[(R)-3-(1-pipéridyl)-3-méthylbutylthio-Sar]³-[4'-hydroxy-MeLeu]⁴-cyclosporine A
[(R)-2-(1-morpholino)éthylthio-Sar]³-[4'-hydroxy-MeLeu]⁴-cyclosporine A ;
[(R)-2-(1-azétidino)éthylthio-Sar]³-[4'-hydroxy-MeLeu]⁴-cyclosporine A ;
{(R)-2-[1-(4-méthylpipérazino)]éthylthio-Sar}³-[4'-hydroxy-MeLeu]⁴-cyclosporine A;
{(R)-2-[1-(4-phénylpipérazino)]éthylthio-Sar}³-[4'-hydroxy-MeLeu]⁴-cyclosporine A;
{(R)-2-[1-(4-benzylpipérazino)]éthylthio-Sar}³-[4'-hydroxy-MeLeu]⁴-cyclosporine A;
{(R)-2-[1-(4-méthyl-1,2,3,6-tétrahydropyridyl)]éthylthio-Sar}³-[4'-hydroxy-MeLeu]⁴-cyclosporine A ;
{(R)-2-[1-(4-phényl-1,2,3,6-tétrahydropyridyl)]éthylthio-Sar}³-[4'-hydroxy-MeLeu]⁴-cyclosporine A ;
[(R)-3-(1-morpholino)propylthio-Sar]³-[4'-hydroxy-MeLeu]⁴-cyclosporine A ;
[(R)-3-(1-azétidino)propylthio-Sar]³-[4'-hydroxy-MeLeu]⁴-cyclosporine A ;
{(R)-3-[1-(4-méthylpipérazino)]propylthio-Sar}³-[4'-hydroxy-MeLeu]⁴-cyclosporine A;
{(R)-3-[1-(4-phénylpipérazino)]propylthio-Sar}³-[4'-hydroxy-MeLeu]⁴-cyclosporine A;
{(R)-3-[1-(4-benzylpipérazino)]propylthio-Sar}³-[4'-hydroxy-MeLeu]⁴-cyclosporine A;
{(R)-3-[1-(4-méthyl-1,2,3,6-tétrahydropyridyl)]propylthio-Sar}³-[4'-hydroxy-MeLeu]⁴-cyclosporine A ;
{(R)-3-[1-(4-méthyl-1,2,3,6-tétrahydropyridyl)]propylthio-Sar}³-[4'-hydroxy-MeLeu]⁴-cyclosporine A.

## Revendications

1. Procédé de préparation d'un polyanion intermédiaire pour la préparation de dérivés de cyclosporine **caractérisé en ce que** l'on traite une cyclosporine par un sel métallique de l'hexaméthyldisilazane, en présence d'un halogénure de métal choisi dans le groupe constitué par le chlorure de lithium, le chlorure de césium, le fluorure de césium, le chlorure cuivreux et le chlorure mercurique, à une température comprise entre -40°C et 0°C.

2. Procédé de préparation d'un polyanion intermédiaire pour la préparation de dérivés de cyclosporine selon la revendication 1, **caractérisé en ce que** le dit polyanion a pour formule : 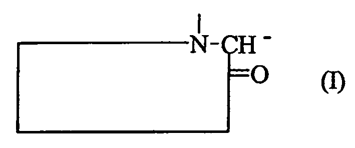 dans laquelle 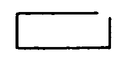 représente une cyclosporine sur laquelle un ou des groupements hydroxy et/ou un ou des atomes d'azote non-méthylés en position α et/ou tout autre groupement acide déprotonable sont éventuellement déprotonés, ou sont sous forme protégée.

3. Procédé selon la revendication 1 **caractérisé en ce que** le dit polyanion a pour formule générale : 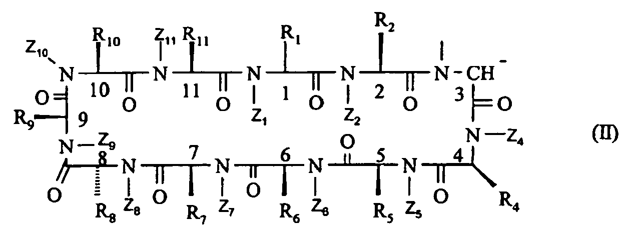 pour laquelle :
i) Les substituants R₁, R₂ et R₄ à R₁₁, et Z₁, Z₂ et Z₄ à Z₁₁ sont définis comme pour la cyclosporine A, ou bien
ii) Les substituants R₁, R₂ et R₄ à R₁₁, et Z₁, Z₂ et Z₄ à Z₁₁ sont définis comme pour la cyclosporine A, à l'exception de R₄ et Z₄ qui sont définis de façon à avoir en position -4 selon la formule (II) ci-dessus, l'acide aminé 4'-(hydroxy)MeLeu, ou bien
iii) Les substituants R₂, R₅ à R₁₁ et Z₂, Z₅ à Z₁₁ sont définis comme pour la cyclosporine A, et
• Z₁ est un groupement méthyle, et R₁ a pour formule : 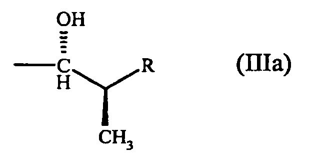
- R étant un groupement de formule -CH₂-CH=CH-CH₂-R' pour lequel R' représente un radical alkylthio, aminoalkylthio, alkylaminoalkylthio, dialkylaminoalkylthio, pyrimidinylthio, thiazolylthio, N-alkylimidazolylthio, hydroxyalkylphénylthio, hydroxyalkylphényloxy, nitrophénylamino, ou 2-oxopyrimidin-1-yle, ou
- R étant un groupement de formule -CH₂-S-Alk pour lequel Alk représente un groupement alkyle, et
• Z₄ et R₄ sont des radicaux tels que l'on a en position -4 selon la formule (II) ci-dessus, un acide aminé MeLeu ou 4'-hydroxyMeLeu, ou bien,
iv)
• Z₁ et R₁ sont des groupements tels que l'on a en position -1 selon la formule (II) ci-dessus, une homothréonine substituée de formule générale :
Rᵢ-CH₂CH(CH₃)-CH(OH)-CH(NHCH₃)-COOH (IIIb)
dans laquelle Rᵢ représente le n-propyle ou le propényle et la double liaison présente de préférence une configuration trans, et
• R₂ et Z₂ sont des radicaux tels que l'on a en position -2 selon la formule (II) ci-dessus, l'acide alpha-aminobutyrique (αAbu), la valine (Val), la norvaline (Nva), ou la thréonine (Thr), et
• R₄ et Z₄ sont des radicaux tels que l'on a en position -4 selon la formule (II) ci-dessus, la N-méthyl-gamma-hydroxy-leucine ou la N-méthyl-gamma-acétyloxyleucine, et
• R₅ et Z₅ sont des radicaux tels que l'on a en position -5 selon la formule (II) ci-dessus, la valine, et
• R₆, Z₆, R₉, Z₉, R₁₀ et Z₁₀ sont des radicaux tels que l'on a en position -6, -9, et - 10 selon la formule (II) ci-dessus, la N-méthylleucine, et
• Z₇ et R₇ sont des radicaux tels que l'on a en position -7 selon la formule (II) ci-dessus, l'alanine (Ala), et
• Z₈ et R₈ sont des radicaux tels que l'on a en position -8 selon la formule (II) ci-dessus, la (D)-alanine ou la (D)-sérine, et
• Z₁₁ et R₁₁ sont des radicaux tels que l'on a en position -11 selon la formule (II) ci-dessus, la N-méthyl-valine, ou bien,
v) Les substituants R₄ à R₁₁, et Z₄ à Z₁₁ sont définis comme pour la cyclosporine A, et : Z₁ et R₁ sont des substituants tels que l'on a en position -1 selon la formule (II) ci-dessus, l'acide aminé MeBmt ou dihydro-MeBmt, et
• Z₂ et R₂ sont des substituants tels que l'on a en position -2 selon la formule (II) ci-dessus, l'acide aminé αAbu, Thr, Val, ou Nva, ou bien
vi) Les substituants R₇ à R₁₁, et Z₇ à Z₁₁ sont définis comme pour la cyclosporine A, et:
• Z₁ et R₁ sont des substituants tels que l'on a en position -1 selon la formule (II) ci-dessus, l'acide aminé MeBmt, dihydro-MeBmt, ou 8'-hydroxy-MeBmt, et
• Z₂ et R₂ sont des substituants tels que l'on a en position -2 selon la formule (II) ci-dessus, l'acide aminé αAbu; Val, Thr, Nva, ou MeOThr, et
• Z₄ et R₄ sont des substituants tels que l'on a en position -4 selon la formule (II) ci-dessus, l'acide aminé MeLeu, γ-hydroxy-MeLeu, MeIle, MeVal, MeThr, MeAla, MeaIle, ou MeaThr, et
• Z₅ et R₅ sont des substituants tels que l'on a en position -5 selon la formule (II) ci-dessus, l'acide aminé Val, Leu, MeVal, ou MeLeu, et
• Z₆ et R₆ sont des substituants tels que l'on a en position -6 selon la formule (II) ci-dessus, l'acide aminé MeLeu, γ-hydroxy-MeLeu, ou MeAla,
à condition que lorsque R₄ et Z₄ signifient MeLeu, R₅ et Z₅ signifient alors MeVal ou MeLeu ou bien R₁ et Z₁ signifient 8'-hydroxy-MeBmt, ou bien
vii) Les substituants R₁, R₂ et R₄ à R₁₁, et Z₁, Z₂ et Z₄ à Z₁₁ définissent une cyclosporine pour laquelle le carbone en 3' du résidu en position -1 selon la formule (II) ci-dessus ou le carbone en β du résidu en position-2 est substitué par O-acyle ou oxo, et notamment
• Z₁ et R₁ sont des substituants tels que l'on a en position -1 selon la formule (II) ci-dessus, un résidu de formule générale 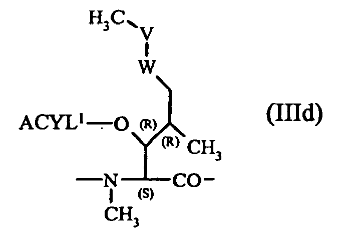 pour laquelle -v-w- est CH₂-CH₂ ou CH=CH trans et ACYL¹ représente un groupe acyle, et
• Z₂ et R₂ sont des substituants tels que l'on a en position -2 selon la formule (II) ci-dessus, un acide aminé αAbu, Val, Thr, Nva, ou le résidu d'un α-acide aminé β-O-acylé, et
• Z₅ et R₅ sont des substituants tels que l'on a en position -5 selon la formule (II) ci-dessus, un acide aminé Val, ou Nva lorsque l'on a simultanément un acide aminé Nva en position -2 selon la formule (II) ci-dessus, et
• Z₈ et R₈ sont des substituants tels que l'on a en position -8 selon la formule (II) ci-dessus, un acide aminé (D)-Ala, un résidu d'un α-acide aminé β-O-acylé ou β-hydroxylé ayant la configuration (D), et
• les substituants en position -4, -6, -7, et -9 à -11 selon la formule (II) ci-dessus sont définis comme pour la cyclosporine A,
un ou des groupements hydroxy et/ou un ou des atomes d'azote non-méthylés en position α et/ou tout autre groupement acide déprotonable présents dans ladite formule générale (II) étant éventuellement déprotonés ou sous forme protégée.

4. Procédé selon les revendications 1 à 3 **caractérisé en ce que** le dit sel métallique de l'hexaméthyldisilazane est un sel alcalin de l'hexaméthyldisilazane.

5. Procédé selon les revendications 1 à 4 **caractérisé en ce que** le dit sel métallique de l'hexaméthyldisilazane est choisi parmi le sel de lithium de l'hexaméthyldisilazane, le sel de sodium de l'hexaméthyldisilazane ou le sel de potassium de l'hexaméthyldisilazane.

6. Procédé selon les revendications 1 à 5 **caractérisé en ce que** en outre, le dit sel métallique de l'hexaméthyldisilazane est utilisé en quantité comprise entre 20 et 30 équivalents molaires.

7. Procédé selon la revendication 1 **caractérisé en ce que**, lorsque ledit halogénure de métal est le chlorure de césium ou de lithium, celui-ci est en outre utilisé en quantité comprise entre 2 et 8 équivalents molaires.

8. Procédé selon l'une quelconque des revendications précédentes, **caractérisé en ce que** en outre, le traitement de la cyclosporine s'effectue dans un éther aliphatique ou cyclique, un hydrocarbure aromatique, ou bien un mélange de ces solvants.

9. Procédé selon l'une quelconque des revendications précédentes, **caractérisé en ce que** en outre, le traitement de la cyclosporine s'effectue avec un ratio (poids/poids) de cyclosporine mise en jeu par rapport au poids total de la solution qui est inférieur ou égal à 10%.

10. Procédé de préparation de dérivés de cyclosporine substitués en position -3, **caractérisé en ce que** l'on prépare un polyanion par traitement d'une cyclosporine par un sel métallique de l'hexaméthyldisilazane en présence d'un halogénure de métal, à une température comprise entre -40°C et 0°C, puis l'on additionne un agent électrophile, les radicaux hydroxy présents sur la cyclosporine pouvant éventuellement interférer avec la réaction étant préalablement protégés, puis l'on élimine le cas échéant les radicaux protecteurs et/ou transforme éventuellement le produit obtenu en sels lorsqu'ils existent.

11. Procédé de préparation selon la revendication 10 **caractérisé en ce que** les dérivés de cyclosporine substitués en position -3 ainsi obtenus ont pour formule générale : 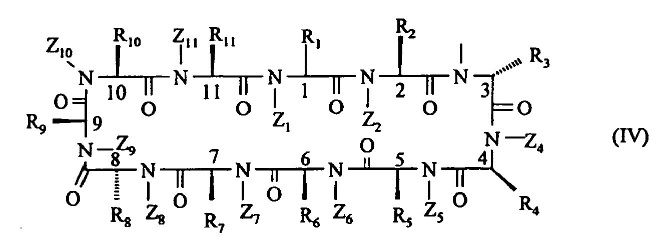 pour laquelle :
1) les substituants R₁ , R₂ et R₄ à R₁₁, et Z₁ , Z₂ et Z₄ à Z₁₁ sont tels que définis dans la revendication 3 en i), et R₃ représente un substituant -S-Alk-R^{o} pour lequel :
- Alk représente un radical alkylène contenant 2 à 6 atomes de carbone en chaîne droite ou ramifiée ou cycloalkylène contenant 3 à 6 atomes de carbone et
- R° représente
• soit un radical carboxy ou alkyloxycarbonyle,
• soit un radical -NG₁G₂ pour lequel G₁ et G₂, identiques ou différents, représentent des atomes d'hydrogène, ou des radicaux alkyle, alcényle (2 à 4C), cycloalkyle (3 à 6C), phényle éventuellement substitué (par un atome d'halogène, alkyloxy, alkyloxycarbonyle, amino, alkylamino ou dialkylamino), ou représentent des radicaux benzyle ou hétérocyclyle saturé ou insaturé contenant 5 ou 6 chaînons et 1 à 3 hétéroatomes, ou pour lequel G₁ et G₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alkyle, phényle ou benzyle,
• soit un radical de formule générale : 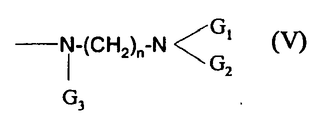 pour lequel G₁ et G₂ sont définis comme ci-dessus, G₃ représente un atome d'hydrogène ou un radical alkyle et n est un nombre entier de 2 à 4, les portions ou radicaux alkyle définis ci-dessus étant droits ou ramifiés et contenant 1 à 4 atomes de carbone, ou bien
2) les substituants R₁, R₂ et R₄ à R₁₁, et Z₁, Z₂ et Z₄ à Z₁₁ sont tels que définis dans la revendication 3 en ii), et R₃ représente -S-CH₃ ou un substituant -S-Alk-R^{o} pour lequel :
- Alk représente un radical alkylène contenant 2 à 6 atomes de carbone en chaîne droite ou ramifiée ou cycloalkylène contenant 3 à 6 atomes de carbone et
- R° représente
• soit un radical hydroxy, carboxy ou alkyloxycarbonyle,
• soit un radical -NG₁G₂ ou un radical de formule générale : 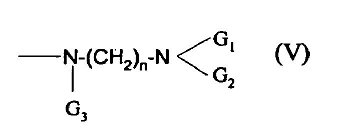 tels que définis ci-avant,
3) les substituants R₁, R₂ et R₄ à R₁₁, et Z₁, Z₂ et Z₄ à Z₁₁ sont tels que définis dans la revendication 3 en iii), et R₃ est un radical de structure -S-Alk-R^{o} pour lequel :
- Alk représente un radical alkylène contenant 2 à 6 atomes de carbone en chaîne droite ou ramifiée ou cycloalkylène contenant 3 à 6 atomes de carbone et
- R° représente
• soit un atome d'hydrogène, un radical hydroxy, carboxy ou alkyloxycarbonyle,
• soit un radical -NG₁G₂ pour lequel G₁ et G₂ identiques ou différents représentent des atomes d'hydrogène, ou des radicaux alkyle, cycloalkyle (3 à 6C), phényle éventuellement substitué (par un atome d'halogène, alkyloxy, alkyloxycarbonyle, amino, alkylamino ou dialkylamino), ou représentent des radicaux benzyle ou hétérocyclyle saturé ou insaturé contenant 5 ou 6 chaînons et 1 à 3 hétéroatomes, ou pour lequel G₁ et G₂ forment avec l'atome d'azote auquel ils sont rattachés un hétérocycle à 5 ou 6 chaînons pouvant contenir un autre hétéroatome choisi parmi l'azote, l'oxygène ou le soufre et éventuellement substitué par alkyl,
• soit un radical de formule générale : 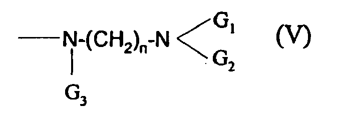 tel que défini précédemment, ou bien
4) les substituants R₁, R₂ et R₄ à R₁₁, et Z₁, Z₂ et Z₄ à Z₁₁ sont tels que définis dans la revendication 3 en iv), et R₃ est un substituant :
- alkyle en C₂ à C₆, alcényle, alcynyle, linéaires ou ramifiés, ces groupes pouvant encore être substitués par un groupe hydroxy, amino, alkylamino en C₁ à C₄, dialkylamino en C₁ à C₃, alkyloxy, ou acyloxy, ou
- COOG₄ ou CONHG₄, G₄ étant un alkyle contenant 1 à 4 atomes de carbone linéaire ou ramifié, ou
- -Y-G₅ dans laquelle Y = S, O, et G₅ est un alkyle en C₁ à C₄, un alcényle ou un alcynyle, linéaires ou ramifiés, et dans laquelle si Y = S, G₅ peut être aussi un aryle ou un hétéroaryle, ou
- un groupement halogène, ou cyano, ou
- CHG₆G₇, dans laquelle G₆ est un atome d'hydrogène, un groupement méthyle, éthyle, phényle, et G₇ est un atome d'hydrogène, ou un groupe hydroxy, halogène, amino, alkylamino en C₁ à C₄, dialkylamino en C₁ à C₄, acyloxy, *t*-butoxycarbonylamino-éthoxy-éthoxy-acétyloxy, ou alkyloxycarbonyle, ou bien
5) Les substituants R₁, R₂ et R₄ à R₁₁, et Z₁, Z₂ et Z₄ à Z₁₁ sont tels que définis dans la revendication 3 en v), et R₃ est un groupement C₁₋₆alkyle, halogénoC₁₋₆alkyle, hydroxyC₁₋₆alkyle, mercaptoC₁₋₆alkyle, amino C₁₋₆alkyle, C₂₋₅alkoxycarbonyl-amino-(C₁₋₄alkyle), nitroC₁₋₆alkyle, cyanoC₁₋₅alkyle, C₁₋₆alkoxy-(C₁₋₆alkyle), C₁₋₆alkylthio-(C₁₋₆alkyle), C₂₋₇alcanoyloxy-(C₁₋₆alkyle), C₂₋₇diazoalcanoyloxy-(C₁₋₆alkyle), carboxy-(C₁₋₆alkyle), C₂₋₇alkoxycarbonyl-(C₁₋₆alkyle), aminocarbonyl-(C₁₋₄alkyle), aminocarbonyloxy-(C₁₋₄alkyle), amino-(C₁₋₄ alcanoyloxy)-(C₁₋₄alkyle), amino-(C₂₋₉alkoxycarbonyl)-(C₁₋₄alkyle), C₂₋₇ alkylcarbonyle, C₂₋₇alkoxycarbonyle, C₁₋₆alkylthio, hydroxy-C₁₋₆alkylthio, C₁₋₆ alkoxy-(C₁₋₆alkylthio), C₂₋₁₁alcanoyloxy-(C₂₋₄alkylthio), C₂₋₁₁alcanoyloxy-(C₂₋₄ alkylsulfinyle), C₂₋₁₁-alcanoyloxy-(C₂₋₄alkylsulfonyle), aminocarbonyloxy-(C₂₋₄ alkylthio), C₂₋₁₁amino-alcanoyloxy-(C₂₋₄alkylthio), aminocarbonyloxy-(C₂₋₄ alkylsulfinyle), aminocarbonyl-oxy-(C₂₋₄alkylsulfonyle), aminoalcanoyloxy-(C₂₋₄ alkylsulfinyle), aminoalcanoyloxy-(C₂₋₄alkylsulfonyle), aminocarbonyle, C₃₋₆ alcényle, C₃₋₆alcynyle, halogénoC₃₋₆alcényle, halogénoC₃₋₆alcynyle, hydroxy C₃₋₆ alcényle, aryl-(C₁₋₆alkyle), aryl-(C₁₋₆alkyle) hydroxylé, aryl-(C₃₋₆alcényle), aryl-(C₃₋₆alcynyle), aryl-(C₃₋₆alcényle) hydroxylé, aryl-(C₃₋₆alcynyle) hydroxylé, arylthio, hétéroarylthio, aryl-(C₂₋₅alkoxycarbonylamino)-(C₁₋₄alkyle), halogène, cyano, ou un groupe de formule Q-(CH₂-CH₂-O)ₙ-CO-O-CH₂- pour laquelle n est 1, 2, ou 3, et Q est amino, ou bien
6) les substituants R₁, R₂ et R₄ à R₁₁, et Z₁, Z₂ et Z₄ à Z₁₁ sont tels que définis dans la revendication 3 en vi), et R₃ est un groupement tel que l'on a en position -3 un acide aminé (D)-MeAla, ou bien
7) les substituants R₁, R₂ et R₄ à R₁₁, et Z₁, Z₂ et Z₄ à Z₁₁ sont tels que définis dans la revendication 3 en vii), et R₃ est un groupement tel que l'on a en position -3 un α-acide aminé N-méthylé en position α et ayant la configuration (D).

## Claims

1. Process for the preparation of an intermediate polyanion for the preparation of cyclosporin derivatives, **characterized in that** a cyclosporin is treated with a metal salt of hexamethyldisilazane, in the presence of a metal halide chosen from the group consisting of lithium chloride, caesium chloride, caesium fluoride, cuprous chloride and mercuric chloride, at a temperature of between -40°C and 0°C.

2. Process for the preparation of an intermediate polyanion for the preparation of cyclosporin derivatives according to Claim 1, **characterized in that** the said polyanion has the formula: 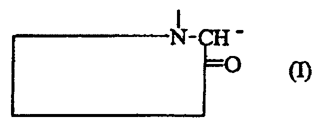 in which 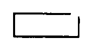 represents a cyclosporine in which one or more hydroxyl groups and/or one or more non-methylated nitrogen atoms at the α position and/or any other deprotonatable acidic group are optionally deprotonated or are in the protected form.

3. Process according to Claim 1, **characterized in that** the said polyanion has the general formula: 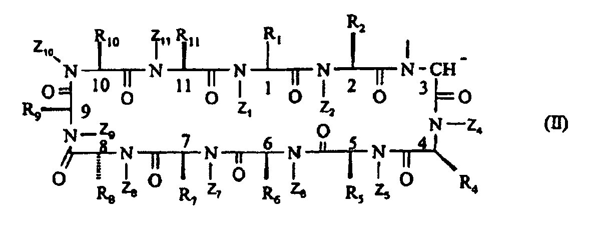 in which:
i) the substituents R₁, R₂ and R₄ to R₁₁ and Z₁, Z₂ and Z₄ to Z₁₁ are defined as for cyclosporin A, or else
ii) the substituents R₁, R₂ and R₄ to R₁₁ and Z₁, Z₂ and Z₄ to Z₁₁ are defined as for cyclosporin A, with the exception of R₄ and Z₄, which are defined so as to have, at the 4-position according to the formula (II) above, the amino acid 4'-hydroxy-MeLeu, or else
iii) the substituents R₂ and R₅ to R₁₁ and Z₂ and Z₅ to Z₁₁ are defined as for cyclosporin A, and
• Z₁ is a methyl group and R₁ has the formula: 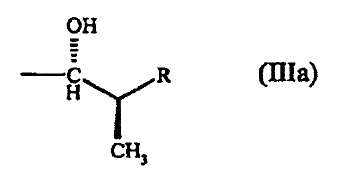
- R being a group of formula -CH₂-CH=CH-CH₂-R' in which R' represents an alkylthio, aminoalkylthio, alkylaminoalkylthio, dialkylaminoalkylthio, pyrimidinylthio, thiazolylthio, N-alkylimidazolylthio, hydroxyalkylphenylthio, hydroxyalkylphenyloxy, nitrophenylamino or 2-oxopyrimidin-1-yl radical, or
- R being a group of formula -CH₂-S-Alk in which Alk represents an alkyl group, and
• Z₄ and R₄ are radicals such that there is, at the 4-position according to the formula (II) above, an amino acid MeLeu or 4'-hydroxy-MeLeu, or else,
iv) Z₁ and R₁ are groups such that there is, at the 1-position according to the formula (II) above, a substituted homothreonine of general formula:
Rᵢ-CH₂CH (CH₃) -CH (OH) -CH (NHCH₃) -COOH (IIIb)
in which Rᵢ represents n-propyl or propenyl and the double bond preferably exhibits a trans configuration, and
• R₂ and Z₂ are radicals such that there is, at the 2-position according to the formula (II) above, α-aminobutyric acid (αAbu), valine (Val), norvaline (Nva) or threonine (Thr), and
• R₄ and Z₄ are radicals such that there is, at the 4-position according to the formula (II) above, N-methyl-γ-hydroxyleucine or N-methyl-γ-acetyloxyleucine, and
• R₅ and Z₅ are radicals such that there is, at the 5-position according to the formula (II) above, valine, and
• R₆, Z₆, R₉, Z₉, R₁₀ and Z₁₀ are radicals such that there is, at the 6-, 9- and 10-position according to the formula (II) above, N-methylleucine, and
• Z₇ and R₇ are radicals such that there is, at the 7-position according to the formula (II) above, alanine (Ala), and
• Z₈ and R₈ are radicals such that there is, at the 8-position according to the formula (II) above, D-alanine or D-serine, and
• Z₁₁ and R₁₁ are radicals such that there is, at the 11-position according to the formula (II) above, N-methylvaline, or else,
v) the substituents R₄ to R₁₁ and Z₄ to Z₁₁ are defined as for cyclosporin A, and:
• Z₁ and R₁ are substituents such that there is, at the 1-position according to the formula (II) above, the amino acid MeBmt or dihydro-MeBmt, and
• Z₂ and R₂ are substituents such that there is, at the 2-position according to the formula (II) above, the amino acid αAbu, Thr, Val or Nva, or else
vi) the substituents R₇ to R₁₁ and Z₇ to Z₁₁ are defined as for cyclosporin A, and:
• Z₁ and R₁ are substituents such that there is, at the 1-position according to the formula (II) above, the amino acid MeBmt, dihydro-MeBmt or 8'-hydroxy-MeBmt, and
• Z₂ and R₂ are substituents such that there is, at the 2-position according to the formula (II) above, the amino acid αAbu, Val, Thr, Nva or MeOThr, and
• Z₄ and R₄ are substituents such that there is, at the 4-position according to the formula (II) above, the amino acid MeLeu, γ-hydroxy-MeLeu, MeIle, MeVal, MeThr, MeAla, MeaIle or MeaThr, and
• Z₅ and R₅ are substituents such that there is, at the 5-position according to the formula (II) above, the amino acid Val, Leu, MeVal or MeLeu, and
• Z₆ and R₆ are substituents such that there is, at the 6-position according to the formula (II) above, the amino acid MeLeu, γ-hydroxy-MeLeu or MeAla,
provided that, when R₄ and Z₄ mean MeLeu, R₅ and Z₅ then mean MeVal or MeLeu or else R₁ and Z₁ mean 8'-hydroxy-MeBmt, or else
vii) the substituents R₁, R₂ and R₄ to R₁₁ and Z₁, Z₂ and Z₄ to Z₁₁ define a cyclosporin in which the 3' carbon of the residue at the 1-position according to the formula (II) above or the β carbon of the residue at the 2-position is substituted by 0-acyl or oxo, and in particular
• Z₁ and R₁ are substituents such that there is, at the 1-position according to the formula (II) above, a residue of general formula 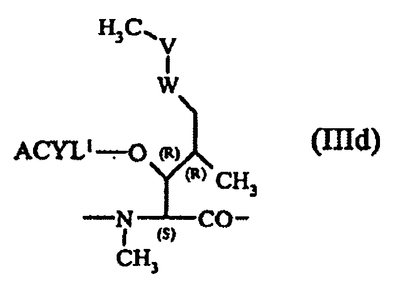 in which -v-w- is CH₂-CH₂ or trans CH=CH and ACYL¹ represents an acyl group, and
• Z₂ and R₂ are substituents such that there is, at the 2-position according to the formula (II) above, an amino acid αAbu, Val, Thr, Nva or a β-O-acylated α-amino acid, and
• Z₅ and R₅ are substituents such that there is, at the 5-position according to the formula (II) above, an amino acid Val or Nva when there is simultaneously an amino acid Nva at the 2-position according to the formula (II) above, and
• Z₈ and R₈ are substituents such that there is, at the 8-position according to the formula (II) above, an amino acid D-Ala or a β-O-acylated or β-hydroxylated α-amino acid having the D configuration, and
• the substituents at the 4-, 6-, 7- and 9- to 11-position according to the formula (II) above are defined as for cyclosporin A,
one or more hydroxyl groups and/or one or more non-methylated nitrogen atoms at the α position and/or any other deprotonatable acidic group present in the said general formula (II) optionally being deprotonated or in the protected form.

4. Process according to Claims 1 to 3, **characterized in that** the said metal salt of hexamethyldisilazane is an alkali metal salt of hexamethyldisilazane.

5. Process according to Claims 1 to 4, **characterized in that** the said metal salt of hexamethyldisilazane is chosen from the lithium salt of hexamethyldisilazane, the sodium salt of hexamethyldisilazane or the potassium salt of hexamethyldisilazane.

6. Process according to Claims 1 to 5, **characterized in that**, in addition, the said metal salt of hexamethyldisilazane is used in an amount of between 20 and 30 molar equivalents.

7. Process according to Claim 1, **characterized in that**, when the said metal halide is caesium chloride or lithium chloride, this is, in addition, used in an amount of between 2 and 8 molar equivalents.

8. Process according to any one of the preceding claims, **characterized in that**, in addition, the treatment of the cyclosporin is carried out in an aliphatic or cyclic ether, an aromatic hydrocarbon or else a mixture of these solvents.

9. Process according to any one of the preceding claims, **characterized in that**, in addition, the treatment of the cyclosporin is carried with a ratio (weight/weight) of cyclosporin involved with respect to the total weight of the solution which is less than or equal to 10%.

10. Process for the preparation of cyclosporin derivatives substituted at the 3-position, **characterized in that** a polyanion is prepared by treatment of a cyclosporin with a metal salt of hexamethyldisilazane in the presence of a metal halide, at a temperature of between -40°C and 0°C, an electrophilic agent is then added, the hydroxyl radicals present on the cyclosporin which may possibly interfere with the reaction being protected beforehand, then, if appropriate, the protective radicals are removed and/or the product obtained is optionally converted to salts, when they exist.

11. Preparation process according to Claim 10, **characterized in that** the cyclosporin derivatives substituted at the 3-position thus obtained have the general formula: 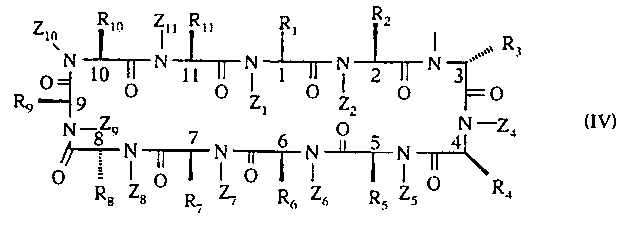 in which:
1) the substituents R₁, R₂ and R₄ to R₁₁ and Z₁, Z₂ and Z₄ to Z₁₁ are as defined in Claim 3 in i) and R₃ represents a substituent -S-Alk-R° in which:
- Alk represents an alkylene radical comprising 2 to 6 straight- or branched-chain carbon atoms or a cycloalkylene radical comprising 3 to 6 carbon atoms and
- R° represents
• either a carboxyl or alkyloxycarbonyl radical,
• or an -NG₁G₂ radical in which G₁ and G₂, which are identical or different, represent hydrogen atoms or optionally substituted (by a halogen atom, alkyloxy, alkyloxycarbonyl, amino, alkylamino or dialkylamino) phenyl, cycloalkyl (3 to 6C), alkenyl (2 to 4C) or alkyl radicals or represent benzyl radicals or saturated or unsaturated heterocyclyl radicals comprising 5 or 6 ring members and 1 to 3 heteroatoms or in which G₁ and G₂ form, with the nitrogen atom to which they are attached, a saturated or unsaturated heterocycle comprising 4 to 6 ring members which can comprise another heteroatom chosen from nitrogen, oxygen or sulphur and which is optionally substituted by alkyl, phenyl or benzyl,
• or a radical of general formula: 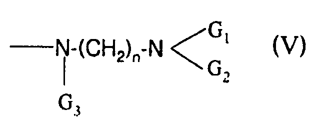 in which G₁ and G₂ are defined as above, G₃ represents a hydrogen atom or an alkyl radical and n is an integer from 2 to 4, the alkyl portions or radicals defined above being straight or branched and comprising 1 to 4 carbon atoms, or else
2) the substituents R₁, R₂ and R₄ to R₁₁ and Z₁, Z₂ and Z₄ to Z₁₁ are as defined in Claim 3 in ii) and R₃ represents -S-CH₃ or a substituent -S-Alk-R^{o} in which:
- Alk represents an alkylene radical comprising 2 to 6 straight- or branched-chain carbon atoms or a cycloalkylene radical comprising 3 to 6 carbon atoms and
- R° represents
• either a hydroxyl, carboxyl or alkyloxycarbonyl radical,
• or an - NG₁G₂ radical or a radical of general formula: 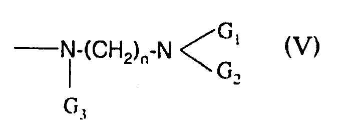 as defined above,
3) the substituents R₁, R₂ and R₄ to R₁₁ and Z₁, Z₂ and Z₄ to Z₁₁ are as defined in Claim 3 in iii) and R₃ is a radical of structure -S-Alk-R^{o} in which:
- Alk represents an alkylene radical comprising 2 to 6 straight- or branched-chain carbon atoms or a cycloalkylene radical comprising 3 to 6 carbon atoms and
- R° represents
• either a hydrogen atom or a hydroxyl, carboxyl or alkyloxycarbonyl radical,
• or an -NG₁G₂ radical in which G₁ and G₂, which are identical or different, represent hydrogen atoms or optionally substituted (by a halogen atom, alkyloxy, alkyloxycarbonyl, amino, alkylamino or dialkylamino) phenyl, cycloalkyl (3 to 6C) or alkyl radicals or represent benzyl radicals or saturated or unsaturated heterocyclyl radicals comprising 5 or 6 ring members and 1 to 3 heteroatoms or in which G₁ and G₂ form, with the nitrogen atom to which they are attached, a 5- or 6-membered heterocycle which can comprise another heteroatom chosen from nitrogen, oxygen or sulphur and which is optionally substituted by alkyl,
• or a radical of general formula: 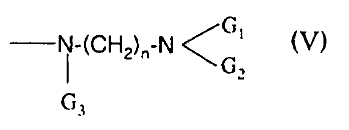 as defined above, or else
4) the substituents R₁, R₂ and R₄ to R₁₁ and Z₁, Z₂ and Z₄ to Z₁₁ are as defined in Claim 3 in iv) and R₃ is a substituent from:
- linear or branched alkyl (C₂ to C₆), alkenyl or alkynyl, it being possible for these groups to be further substituted by a hydroxyl, amino, C₁ to C₄ alkylamino, C₁ to C₃ dialkylamino, alkyloxy or acyloxy group, or
- COOG₄ or CONHG₄, G₄ being a linear or branched alkyl comprising 1 to 4 carbon atoms, or
- -Y-G₅, in which Y = S or O and G₅ is a linear or branched C₁ to C₄ alkyl, a linear or branched alkenyl or a linear or branched alkynyl and in which, if Y = S, G₅ can also be an aryl or a heteroaryl, or
- a halo or cyano group, or
- CHG₆G₇, in which G₆ is a hydrogen atom or a methyl, ethyl or phenyl group and G₇ is a hydrogen atom or a hydroxyl, halo, amino, C₁ to C₄ alkylamino, C₁ to C₄ dialkylamino, acyloxy, *t*-butoxycarbonylaminoethoxyethoxyacetyloxy or alkyloxycarbonyl group, or else
5) the substituents R₁, R₂ and R₄ to R₁₁ and Z₁, Z₂ and Z₄ to Z₁₁ are as defined in Claim 3 in v) and R₃ is a C₁₋₆alkyl, haloC₁₋₆alkyl, hydroxyC₁₋₆alkyl, mercaptoC₁₋₆alkyl, aminoC₁₋₆alkyl, C₂₋₅alkoxycarbonylamino(C₁₋₄alkyl), nitroC₁₋₆alkyl, cyanoC₁₋₅alkyl, C₁₋₆alkoxy(C₁₋₆alkyl), C₁₋₆alkylthio-(C₁₋₆alkyl), C₂₋₇alkanoyloxy(C₁₋₆alkyl), C₂₋₇diazoalkanoyloxy(C₁₋₆alkyl), carboxy(C₁₋₆alkyl), C₂₋₇alkoxycarbonyl (C₁₋₆alkyl), aminocarbonyl(C₁₋₄alkyl), aminocarbonyloxy(C₁₋₄alkyl), amino (C₁₋₄alkanoyloxy) (C₁₋₄alkyl), amino (C₂₋₉alkoxycarbonyl) (C₁₋₄alkyl), C₂₋₇alkylcarbonyl, C₂₋₇alkoxycarbonyl, C₁₋₆alkylthio, hydroxyC₁₋₆alkylthio, C₁₋₆alkoxy (C₁₋₆alkylthio), C₂₋₁₁alkanoyloxy (C₂₋₄alkylthio), C₂₋₁₁alkanoyloxy(C₂₋₄alkylsulphinyl), C₂₋₁₁alkanoyloxy (C₂₋₄alkylsulphonyl), aminocarbonyloxy (C₂₋₄alkylthio), C₂₋₁₁aminoalkanoyloxy(C₂₋₄alkylthio), aminocarbonyloxy (C₂₋₄alkylsulphinyl), aminocarbonyloxy(C₂₋₄alkylsulphonyl), aminoalkanoyloxy(C₂₋₄alkylsulphinyl), aminoalkanoyloxy(C₂₋₄alkylsulphonyl), aminocarbonyl, C₃₋₆alkenyl, C₃₋₆alkynyl, haloC₃₋₆alkenyl, haloC₃₋₆alkynyl, hydroxyC₃₋₆alkenyl, aryl(C₁₋₆alkyl), hydroxylated aryl(C₁₋₆alkyl), aryl(C₃₋₆alkenyl), aryl(C₃₋₆alkynyl), hydroxylated aryl (C₃₋₆alkenyl), hydroxylated aryl(C₃₋₆alkynyl), arylthio, heteroarylthio, aryl(C₂₋₅alkoxycarbonylamino)(C₁₋₄alkyl), halo or cyano group or a group of formula Q-(CH₂-CH₂-O)ₙ-CO-O-CH₂- in which n is 1, 2 or 3 and Q is amino, or else,
6) the substituents R₁, R₂ and R₄ to R₁₁ and Z₁, Z₂ and Z₄ to Z₁₁ are as defined in Claim 3 in vi) and R₃ is a group such that there is, at the 3-position, an amino acid D-MeAla, or else
7) the substituents R₁, R₂ and R₄ to R₁₁ and Z₁, Z₂ and Z₄ to Z₁₁ are as defined in Claim 3 in vii) and R₃ is a group such that there is, at the 3-position, an α-amino acid which is N-methylated at the α position and which has the D configuration.

## Patentansprüche

1. Verfahren zur Herstellung eines Polyanion-Zwischenprodukts zur Herstellung von Cyclosporinderivaten, **dadurch gekennzeichnet, dass** man ein Cyclosporin mit einem Metallsalz von Hexamethyldisilazan in Gegenwart eines Metallhalogenids, das ausgewählt ist aus der Gruppe, bestehend aus Lithiumchlorid, Cäsiumchlorid, Cäsiumfluorid, Kupferchlorid und Quecksilberchlorid, bei einer Temperatur zwischen -40°C und 0°C behandelt.

2. Verfahren zur Herstellung eines Polyanion-Zwischenprodukts zur Herstellung von Cyclosporinderivaten nach Anspruch 1, **dadurch gekennzeichnet, dass** das Polyanion folgende Formel hat: 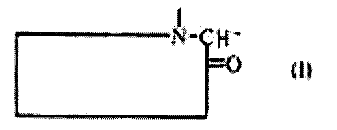 worin 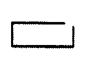 für ein Cyclosporin steht, an dem eine oder mehrere Hydroxygruppen und/oder ein oder mehrere unmethylierte Stickstoffatome an der α-Position und/oder jede andere deprotonierbare Säuregruppe gegebenenfalls deprotoniert ist/sind oder in geschützter Form vorliegen.

3. Verfahren nach Anspruch 1, **dadurch gekennzeichnet, dass** das Polyanion folgende allgemeine Formel hat: 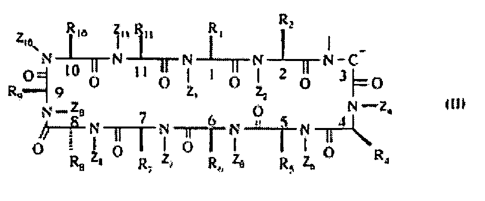 worin:
i) die Substituenten R₁, R₂ und R₄ bis R₁₁ und Z₁, Z₂ und Z₄ bis Z₁₁ wie für Cyclosporin A definiert sind, oder
ii) die Substituenten R₁, R₂ und R₄ bis R₁₁ und Z₁, Z₂ und Z₄ bis Z₁₁ wie für Cyclosporin A definiert sind, mit Ausnahme von R₄ und Z₄, die so definiert sind, dass sie an Position -4 gemäß der vorstehenden Formel (II) die Aminosäure 4'-(Hydroxy)-MeLeu aufweisen, oder
iii) die Substituenten R₂, R₅ bis R₁₁ und Z₂, Z₅ bis Z₁₁ wie für Cyclosporin A definiert sind, und
• Z₁ eine Methylgruppe ist und R₁ folgende Formel hat: 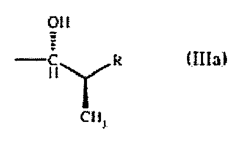
- wobei R eine Gruppe der Formel -CH₂-CH=CH-CH₂-R' ist, wobei R' einen Alkylthio-, Aminoalkylthio-, Alkylaminoalkylthio-, Dialkylaminoalkylthio-, Pyrimidinylthio-, Thiazolylthio-, N-Alkylimidazolylthio-, Hydroxyalkylphenylthio-, Hydroxyalkylphenyloxy-, Nitrophenylamino- oder 2-Oxopyrimidin-1-ylrest darstellt, oder
- R eine Gruppe der Formel -CH₂-S-Alk ist, wobei Alk für eine Alkylgruppe steht, und
• Z₄ und R₄ derartige Reste sind, dass man an Position -4 gemäß der vorstehenden Formel (II) eine Aminosäure MeLeu oder 4'-Hydroxy-MeLeu erhält, oder
iv)
• Z₁ und R₁ derartige Gruppen sind, dass man an Position -1 gemäß der vorstehenden Formel (II) ein substituiertes Homothreonin der folgenden allgemeinen Formel erhält:
Rᵢ-CH₂CH(CH₃)-CH(OH)-CH(NHCH₃)-COOH (IIIb)
worin Rᵢ für n-Propyl oder Propenyl steht und die Doppelbindung vorzugsweise eine trans-Konfiguration aufweist, und
• R₂ und Z₂ derartige Reste sind, dass man an Position -2 gemäß der vorstehenden Formel (II) alpha-Aminobuttersäure (αAbu), Valin (Val), Norvalin (Nva) oder Threonin (Thr) erhält, und
• R₄ und Z₄ derartige Reste sind, dass man an Position -4 gemäß der vorstehenden Formel (II) N-Methylgamma-hydroxyleucin oder N-Methyl-gamma-acetyloxyleucin erhält, und
• R₅ und Z₅ derartige Reste sind, dass man an Position -5 gemäß der vorstehenden Formel (II) Valin erhält, und
• R₆, Z₆, R₉, Z₉, R₁₀ und Z₁₀ derartige Reste sind, dass man an Position -6, -9, und -10 gemäß der vorstehenden Formel (II) N-Methylleucin erhält, und
• Z₇ und R₇ derartige Reste sind, dass man an Position -7 gemäß der vorstehenden Formel (II) Alanin (Ala) erhält, und
• Z₈ und R₈ derartige Reste sind, dass man an Position -8 gemäß der vorstehenden Formel (II) (D)-Alanin oder (D)-Serin erhält, und
• Z₁₁ und R₁₁ derartige Reste sind, dass man an Position -11 gemäß der vorstehenden Formel (II) N-Methylvalin erhält, oder
v) die Substituenten R₄ bis R₁₁ und Z₄ bis Z₁₁ wie für Cyclosporin A definiert sind und:
• Z₁ und R₁ derartige Substituenten sind, dass man an Position -1 gemäß der vorstehenden Formel (II) die Aminosäure MeBmt oder Dihydro-MeBmt erhält, und
• Z₂ und R₂ derartige Substituenten sind, dass man an Position -2 gemäß der vorstehenden Formel (II) die Aminosäure αAbu, Thr, Val oder Nva erhält, oder
vi) die Substituenten R₇ bis R₁₁ und Z₇ bis Z₁₁ wie für Cyclosporin A definiert sind und:
• Z₁ und R₁ derartige Substituenten sind, dass man an Position -1 gemäß der vorstehenden Formel (II) die Aminosäure MeBmt, Dihydro-MeBmt oder 8'-Hydroxy-MeBmt erhält, und
• Z₂ und R₂ derartige Substituenten sind, dass man an Position -2 gemäß der vorstehenden Formel (II) die Aminosäure αAbu, Val, Thr, Nva oder MeOThr erhält, und
• Z₄ und R₄ derartige Substituenten sind, dass man an Position -4 gemäß der vorstehenden Formel (II) die Aminosäure MeLeu, γ-Hydroxy-MeLeu, MeIle, MeVal, MeThr, MeAla, MeaIle oder MeaThr erhält, und
• Z₅ und R₅ derartige Substituenten sind, dass man an Position -5 gemäß der vorstehenden Formel (II) die Aminosäure Val, Leu, MeVal oder MeLeu erhält, und
• Z₆ und R₆ derartige Substituenten sind, dass man an Position -6 gemäß der vorstehenden Formel (II) die Aminosäure MeLeu, γ-Hydroxy-MeLeu oder MeAla erhält,
unter der Bedingung, dass, wenn R₄ und Z₄ MeLeu bedeuten, R₅ und Z₅ dann MeVal oder MeLeu bedeuten oder R₁ und Z₁ 8'-Hydroxy-MeBmt bedeuten, oder
vii) die Substituenten R₁, R₂ und R₄ bis R₁₁ und Z₁, Z₂ und Z₄ bis Z₁₁ ein Cyclosporin definieren, worin das Kohlenstoffatom 3' von dem Rest an Position -1 gemäß der vorstehenden Formel (II) oder das Kohlenstoffatom β von dem Rest an Position -2 mit 0-Acyl oder Oxo substituiert ist, und insbesondere
• Z₁ und R₁ derartige Substituenten sind, dass man an Position -1 gemäß der vorstehenden Formel (II) einen Rest der folgenden allgemeinen Formel erhält 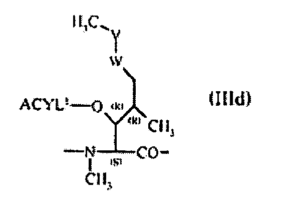 worin -v-w- CH₂-CH₂ oder CH=CH trans ist und ACYL¹ für eine Acylgruppe steht, und
• Z₂ und R₂ derartige Substituenten sind, dass man an Position -2 gemäß der vorstehenden Formel (II) eine Aminosäure αAbu, Val, Thr, Nva oder eine β-O-acylierte α-Aminosäure erhält, und
• Z₅ und R₅ derartige Substituenten sind, dass man an Position -5 gemäß der vorstehenden Formel (II) eine Aminosäure Val oder Nva erhält, wenn gleichzeitig eine Aminosäure Nva an Position -2 gemäß der vorstehenden Formel (II) vorliegt, und
• Z₈ und R₈ derartige Substituenten sind, dass man an Position -8 gemäß der vorstehenden Formel (II) eine Aminosäure (D)-Ala, eine β-O-acylierte oder βhydroxylierte α-Aminosäure mit der (D)-Konfiguration erhält, und
• die Substituenten an Position -4, -6, -7, und -9 bis -11 gemäß der vorstehenden Formel (II) wie für Cyclosporin A definiert sind,
wobei eine oder mehrere Hydroxygruppen und/oder ein oder mehrere unmethylierte Stickstoffatome an der α-Position und/oder jede andere deprotonierbare Säuregruppe, die in der allgemeinen Formel (II) vorliegen, gegebenenfalls deprotoniert sind oder in geschützter Form vorliegen.

4. Verfahren nach den Ansprüchen 1 bis 3, **dadurch gekennzeichnet, dass** das Metallsalz von Hexamethyldisilazan ein Alkalisalz von Hexamethyldisilazan ist.

5. Verfahren nach den Ansprüchen 1 bis 4, **dadurch gekennzeichnet, dass** das Metallsalz von Hexamethyldisilazan ausgewählt ist aus dem Lithiumsalz von Hexamethyldisilazan, dem Natriumsalz von Hexamethyldisilazan oder dem Kaliumsalz von Hexamethyldisilazan.

6. Verfahren nach den Ansprüchen 1 bis 5, **dadurch gekennzeichnet, dass** das Metallsalz von Hexamethyldisilazan ferner in einer Menge zwischen 20 und 30 Moläquivalenten verwendet wird.

7. Verfahren nach Anspruch 1, **dadurch gekennzeichnet, dass**, wenn das Metallhalogenid Cäsiumchlorid oder Lithiumchlorid ist, dieses außerdem in einer Menge zwischen 2 und 8 Moläquivalenten verwendet wird.

8. Verfahren nach einem vorhergehenden Anspruch, **dadurch gekennzeichnet, dass** die Behandlung von Cyclosporin außerdem in einem aliphatischen oder cyclischen Ether, einem aromatischen Kohlenwasserstoff oder auch in einem Gemisch dieser Lösungsmittel erfolgt.

9. Verfahren nach einem vorhergehenden Anspruch, **dadurch gekennzeichnet, dass** die Behandlung von Cyclosporin außerdem mit einem Verhältnis (w/w) von eingesetztem Cyclosporin, bezogen auf das Gesamtgewicht der Lösung, von weniger oder gleich 10% erfolgt.

10. Verfahren zur Herstellung von Cyclosporinderivaten die an Position -3 substituiert sind, **dadurch gekennzeichnet, dass** man ein Polyanion durch Behandlung eines Cyclosporins mit einem Metallsalz von Hexamethyldisilazan in Gegenwart eines Metallhalogenids bei einer Temperatur zwischen -40°C und 0°C herstellt und anschließend ein elektrophiles Mittel zugibt, wobei die Hydroxyreste, die am Cyclosporin vorliegen und gegebenenfalls die Umsetzung stören können, zuvor geschützt werden, und dann gegebenenfalls die Schutzgruppen entfernt und/oder das erhaltene Produkt gegebenenfalls in Salze umwandelt, wenn diese existieren.

11. Verfahren zur Herstellung nach Anspruch 10, **dadurch gekennzeichnet, dass** die so erhaltenen Cyclosporinderivate, die an Position -3 substituiert sind, folgende allgemeine Formel haben: 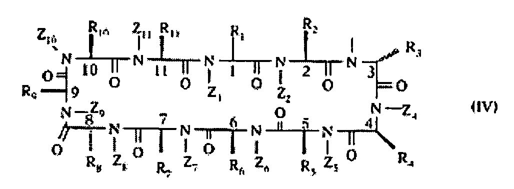 worin:
1) die Substituenten R₁, R₂ und R₄ bis R₁₁ und Z₁, Z₂ und Z₄ bis Z₁₁ wie im Anspruch 3 i) definiert sind und R₃ einen Substituenten -S-Alk-R° darstellt, wobei:
- Alk für einen Alkylenrest steht, der 2 bis 6 Kohlenstoffatome in einer geraden oder verzweigten Kette enthält, oder für ein Cycloalkylen mit 3 bis 6 Kohlenstoffatomen und
- R° folgendes bedeutet
• entweder einen Carboxy- oder Alkyloxycarbonylrest,
• oder einen Rest -NG₁G₂, wobei G₁ und G₂, die gleich oder verschieden sind, Wasserstoffatome oder Alkyl-, (C₂-₄)-Alkenyl-, (C₃-₆) -Cycloalkyl-, gegebenenfalls (mit einem Halogenatom, Alkyloxy, Alkyloxycarbonyl, Amino, Alkylamino oder Dialkylamino) substituierte Phenylreste darstellen oder gesättigte oder ungesättigte Benzyl- oder Heterocyclylreste darstellen, die 5 oder 6 Ringglieder und 1 bis 3 Heteroatome enthalten, oder wobei G₁ und G₂ mit dem Stickstoffatom, an das sie gebunden sind, einen gesättigten oder ungesättigten Heterocyclus mit 4 bis 6 Ringgliedern bilden, der ein weiteres Heteroatom enthalten kann, das ausgewählt ist aus Stickstoff, Sauerstoff oder Schwefel, und der gegebenenfalls mit Alkyl, Phenyl oder Benzyl substituiert ist,
• oder einen Rest der folgenden allgemeinen Formel: 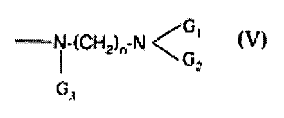 wobei G₁ und G₂ wie vorstehend definiert sind, G₃ ein Wasserstoffatom oder einen Alkylrest darstellt und n eine ganze Zahl von 2 bis 4 ist, wobei die vorstehend definierten Alkylanteile oder -reste gerade oder verzweigt sind und 1 bis 4 Kohlenstoffatome enthalten, oder
2) die Substituenten R₁, R₂ und R₄ bis R₁₁ und Z₁, Z₂ und Z₄ bis Z₁₁ wie im Anspruch 3 ii) definiert sind und R₃ für -S-CH₃ oder einen Substituenten -S-Alk-R° steht, wobei:
- Alk für einen Alkylenrest steht, der 2 bis 6 Kohlenstoffatome in einer geraden oder verzweigten Kette enthält, oder für ein Cycloalkylen mit 3 bis 6 Kohlenstoffatomen und
- R° folgendes darstellt
• entweder einen Hydroxy-, Carboxy- oder Alkyloxycarbonylrest,
• oder einen Rest -NG₁G₂ oder einen Rest der folgenden allgemeinen Formel: 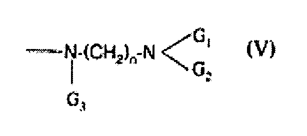 wie vorstehend definiert,
3) die Substituenten R₁, R₂ und R₄ bis R₁₁ und Z₁, Z₂ und Z₄ bis Z₁₁ wie im Anspruch 3 iii) definiert sind und R₃ ein Rest der Struktur -S-Alk-R° ist, wobei:
- Alk für einen Alkylenrest steht, der 2 bis 6 Kohlenstoffatome in einer geraden oder verzweigten Kette enthält, oder für ein Cycloalkylen mit 3 bis 6 Kohlenstoffatomen und
- R° folgendes darstellt
• entweder ein Wasserstoffatom, einen Hydroxy-, Carboxy- oder Alkyloxycarbonylrest,
• oder einen Rest -NG₁G₂, wobei G₁ und G₂, die gleich oder verschieden sind, Wasserstoffatome oder Alkyl-, (C₃-₆)-Cycloalkyl-, gegebenenfalls (mit einem Halogenatom, Alkyloxy, Alkyloxycarbonyl, Amino, Alkylamino oder Dialkylamino) substituierte Phenylreste darstellen oder gesättigte oder ungesättigte Benzyl- oder Heterocyclylreste darstellen, die 5 oder 6 Ringglieder und 1 bis 3 Heteroatome enthalten, oder wobei G₁ und G₂ mit dem Stickstoffatom, an das sie gebunden sind, einen Heterocyclus mit 5 bis 6 Ringgliedern bilden, der ein weiteres Heteroatom enthalten kann, das ausgewählt ist aus Stickstoff, Sauerstoff oder Schwefel, und der gegebenenfalls mit Alkyl substituiert ist,
• oder einen Rest der folgenden allgemeinen Formel: 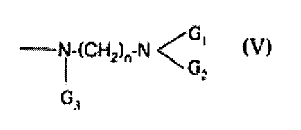 wie vorstehend definiert, oder
4) die Substituenten R₁, R₂ und R₄ bis R₁₁ und Z₁, Z₂ und Z₄ bis Z₁₁ wie im Anspruch 3 iv) definiert sind und R₃ folgender Substituent ist:
- gerades oder verzweigtes C₂-C₆-Alkyl, Alkenyl, Alkinyl, wobei diese Gruppen weiter mit einer Hydroxy-, Amino-, C₁-C₄-Alkylamino-, C₁-C₃-Dialkylamino-, Alkoxy- oder Acyloxygruppe substituiert sein können, oder
- COOG₄ oder CONHG₄, wobei G₄ ein Alkyl mit 1 bis 4 geraden oder verzweigten Kohlenstoffatomen ist, oder
- -Y-G₅, wobei Y = S, O ist und G₅ ein C₁-C₄-Alkyl, ein Alkenyl oder ein Alkinyl ist, die gerade oder verzweigt sind, und wobei, wenn Y = S, G₅ auch ein Aryl oder ein Heteroaryl sein kann, oder
- eine Halogen- oder Cyanogruppe, oder
- CHG₆G₇, wobei G₆ ein Wasserstoffatom, eine Methyl-, Ethyl-, Phenylgruppe ist, und G₇ ein Wasserstoffatom oder eine Hydroxy-, Halogen-, Amino-, C₁-C₄-Alkylamino-, C₁-C₄-Dialkylamino-, Acyloxy-, tert.-Butoxycarbonyl-amino-ethoxy-ethoxy-acetyloxy oder Alkyloxycarbonylgruppe ist, oder
5) die Substituenten R₁, R₂ und R₄ bis R₁₁ und Z₁, Z₂ und Z₄ bis Z₁₁ wie im Anspruch 3 v) definiert sind und R₃ eine C₁₋₆-Alkyl-, Halogen-C₁₋₆-alkyl-, HydroxyC₁₋₆-alkyl-, Mercapt0C₁₋₆-alkyl-, AminoC₁₋₆-alkyl-, C₂₋₅-Alkoxycarbonylamino-(C₁₋₄-alkyl)-, Nitro-C₁₋₆-alkyl-, CyanoC₁₋₅-alkyl-, C₁₋₆-Alkoxy- (C₁₋₆-alkyl) -, C₁₋₆-Alkylthio- (C₁₋₆-alkyl)-, C₂₋₇-Alkanoyloxy- (C₁₋₆-alkyl) -, C₂₋₇-Diazoalkanoyloxy- (C₁₋₆-alkyl) -, Carboxy- (C₁₋₆-alkyl) -, C₂₋₇-Alkoxycarbonyl-(C₁₋₆-alkyl)-, Aminocarbonyl-(C₁₋₄-alkyl)-, Aminocarbonyloxy- (C₁₋₄-alkyl) -, Amino- (C₁₋₄-alkanoyloxy) - (C₁₋₄-alkyl) -, Amino-(C₂₋₉-alkoxycarbonyl)-(C₁₋₄-alkyl)-, C₂-₇-Alkylcarbonyl-, C₂₋₇-Alkoxycarbonyl-, C₁₋₆-Alkylthio-, Hydroxy-C₁₋₆-alkylthio-, C₁₋₆-Alkoxy-(C₁₋₆-alkylthio)-, C₂₋₁₁-Alkanoyloxy- (C₂₋₄-alkylthio) -, C₂₋₁₁-Alkanoyloxy- (C₂-₄-alkylsulfinyl) -, C₂₋₁-Alkanoyloxy- (C₂₋₄-alkylsulfonyl) -, Aminocarbonyloxy-(C₂₋₄-alkylthio)-, C₂₋₁₁-Aminoalkanoyloxy-(C₂₋₄-alkylthio)-, Aminocarbonyloxy-(C₂₋₄-alkylsulfinyl)-, Aminocarbonyloxy-(C₂₋₄-alkylsulfonyl)-, Aminoalkanoyloxy-(C₂₋₄-alkylsulfinyl)-, Aminoalkanoyloxy-(C₂₋₄-alkylsulfonyl)-, Aminocarbonyl-, C₃₋₆-Alkenyl-, C₃₋₆-Alkinyl-, Halogen-C₃₋₆-alkenyl-, Halogen-C₃₋₆-alkinyl-, Hydroxy-C₃₋₆-alkenyl-, Aryl (C₁₋₆-alkyl) -, Hydroxylaryl-(C₁₋₆-alkyl)-, Aryl-(C₃₋₆-alkenyl)-, Aryl(C₃₋₆-alkinyl)-, Hydroxyaryl-(C₃₋₆-alkenyl)-, Hydroxyaryl-(C₃₋₆-alkinyl)-, Arylthio-, Heteroarylthio-, Aryl-(C₂₋₅-alkoxycarbonylamino)-(C₁₋₄-alkyl)-, Halogen-, Cyanogruppe oder einer Gruppe der Formel Q- (CH₂-CH₂-O)ₙ-CO-O-CH₂-, wobei n gleich 1, 2 oder 3 ist und Q ein Amino ist, oder
6) die Substituenten R₁, R₂ und R₄ bis R₁₁ und Z₁, Z₂ und Z₄ bis Z₁₁ wie im Anspruch 3 vi) definiert sind und R₃ eine derartige Gruppe ist, dass man an Position -3 eine Aminosäure (D)-MeAla erhält, oder
7) die Substituenten R₁, R₂ und R₄ bis R₁₁ und Z₁, Z₂ und Z₄ bis Z₁₁ wie im Anspruch 3 vii) definiert sind und R₃ eine derartige Gruppe ist, dass man an Position -3 eine an der α-Position N-methylierte α-Aminosäure mit der (D)-Konfiguration erhält.
